# EUROPÄISCHE PATENTSCHRIFT

(11) **EP 1 806 330 B1**
(45) Veröffentlichungstag und Bekanntmachung des Hinweises auf die Patenterteilung: **14.08.2013**
(21) Anmeldenummer: 06123980.2
(22) Anmeldetag: 14.11.2006
(51) Int. Cl.: C07C 7/04

(54) **Verfahren zur Herstellung von 1-Buten aus technischen Mischungen von C4-Kohlenwasserstoffen**
Process for the preparation of 1-butene from technical mixtures of C4-hydrocarbons
Procédé pour la production de 1-butène à partir de mélanges techniques de C4-hydrocarbures

(30) Priorität: 28.12.2005 DE 102005062700
(43) Veröffentlichungstag der Anmeldung: 11.07.2007
(73) Patentinhaber: Evonik Oxeno GmbH, 45772 Marl (DE)
(72) Erfinder: Rix, Armin, 45770, Marl (DE); Peters, Udo, 45770, Marl (DE); Praefke, Jochen, 45739, Oer-Erkenschwick (DE); Röttger, Dirk, 45657, Recklinghausen (DE); Nierlich, Franz, 45768, Marl (DE)
(74) Vertreter: Hirsch, Hans-Ludwig

(56) Entgegenhaltungen:
- DE-B3- 10 302 457
- US-A- 4 797 133

## Beschreibung

Die Erfindung betrifft ein Verfahren zur Herstellung von 1-Buten aus technischen Mischungen von C₄-Kohlenwasserstoffen, die mindestens 1-Buten, Isobuten, n-Butan und 2-Butene aufweisen.

1-Buten wird, neben anderen C₄-Kohlenwasserstoffen wie Isobuten und 2-Butenen, in großen Mengen aus technischen C₄-Schnitten, beispielsweise dem C₄-Schnitt aus Steamcrackern oder FCC-Einheiten, gewonnen. Diese C₄-Schnitte bestehen im Wesentlichen aus Butadien, den Monoolefinen Isobuten, 1-Buten und den beiden 2-Butenen sowie den gesättigten Kohlenwasserstoffen Isobutan und n-Butan. Wegen der geringen Siedepunktsunterschiede der Inhaltsstoffe und deren geringen Trennfaktoren ist eine destillative Aufarbeitung schwierig und nicht wirtschaftlich. Die Gewinnung von linearen Butenen und von anderen Produkten erfolgt daher meistens durch eine Kombination von chemischen Umsetzungen und physikalischen Trennoperationen.

Der erste Schritt, den standardmäßig alle Aufarbeitungsvarianten gemeinsam haben, ist die Entfernung des größten Teils des enthaltenen Butadiens. Dies wird entweder mit Hilfe einer Extraktivdestillation abgetrennt oder selektiv zu Butenen hydriert. Zurück bleibt in beiden Fällen ein Kohlenwasserstoffgemisch (sogenanntes Raffinat I oder hydriertes Crack-C₄), das neben den gesättigten Kohlenwasserstoffen n-Butan und Isobutan die Olefine Isobuten, 1-Buten und 2-Butene (cis und trans) enthält und in dem mehrfach ungesättigte C₄-Kohlenwasserstoffe typischer Weise in unter 1 % Anteil vorliegen.

Aufgrund der eng beieinanderliegenden Siedepunkte von 1-Buten und Isobuten kann auch aus diesen Mischungen kein 1-Buten über einfache Destillation wirtschaftlich abgetrennt werden. Aus Raffinat I oder hydriertem Crack-C₄ wird daher durch eine selektive chemische Umsetzung Isobuten möglichst weitgehend entfernt. Nach der Entfernung des Isobutens bleibt ein Kohlenwasserstoffgemisch (Raffinat II), das die linearen Butene und die gesättigten Kohlenwasserstoffe Isobutan und n-Butan enthält. Dieses Gemisch kann destillativ weiter aufgetrennt werden, beispielsweise in Isobutan und 1-Buten und ein Gemisch aus den beiden 2-Butenen und n-Butan. Aus der 1-Buten-haltigen Fraktion kann in weiteren Destillationsschritten 1-Buten in hoher Reinheit erhalten werden, welches nur noch geringe Mengen an Isobuten enthält. Dies ist notwendig, da 1-Buten im großen Maße als Comonomer in der Ethylenpolymerisation eingesetzt wird, wo Isobuten-Verunreinigungen unerwünscht sind. Typische Spezifikationen von 1-Buten beschränken daher den Gehalt an Isobuten im 1-Buten auf unter 2000 ppm.

Für die selektive chemische Umsetzung des Isobutens sind verschiedene Verfahren bekannt. Eine Möglichkeit zur Abtrennung von Isobuten ist die Umsetzung mit Alkoholen, beispielsweise Methanol oder Ethanol, zu den entsprechenden tertiären Butylethern. Der Vorteil dieser Umsetzung besteht darin, dass das Isobuten in Gegenwart von linearen Butenen mit hoher Selektivität nahezu vollständig umgesetzt werden kann (ohne dass ein merklicher Umsatz an n-Butenen auftritt). Hierzu wurden diverse verfahrenstechnische Varianten entwickelt. Die Technik der Reaktivdestillation hat sich dabei als besonders nützlich zur Ereichung hoher Isobuten-Umsätze erwiesen.

Das technisch bedeutendste Verfahren ist die Umsetzung von Isobuten mit Methanol zu Methyl-tert.-butylether (MTBE), das hauptsächlich als Kraftstoffzusatz große Verwendung findet.

Eine weitere Möglichkeit der chemische Umsetzung des Isobutens besteht in der Umsetzung mit Wasser zu tert.-Butylalkohol (TBA). Dieser Weg ist wegen der geringen Löslichkeit von Wasser in C₄-Kohlenwasserstoffen technisch aufwändiger als die Ethersynthese.

Eine andere Möglichkeit besteht darin, das Isobuten zu oligomerisieren und das Oligomerisat abzutrennen. Nachteilig dabei ist, dass bei der vollständigen Isobuten-Entfernung durch Oligomerisation auch ein großer Teil der vorliegenden linearen Butene zu Co- oder Homooligomeren umgesetzt wird. Ein weiterer Nachteil ist die teilweise Isomerisierung von 1-Buten zu den 2-Butenen.

Eine weitere Möglichkeit der chemischen Umsetzung des Isobutens in Gegenwart von anderen C₄-Kohlenwasserstoffen ist die Umsetzung mit Formaldehyd. Die dabei erhaltenen Produkte werden beispielsweise zu Isopren weiterverarbeitet.

Viele der bekannten Umsetzungen des Isobutens, beispielsweise zu tert.-Butylalkohol (TBA) oder Isobutenoligomeren, liefern keinen vollständigen Isobuten-Umsatz oder bei hohen Umsätzen in Gegenwart von linearen Butenen nur schlechte Selektivitäten. Als eine Lösung wird beispielsweise eine Kombination dieser Prozesse mit einer gleichzeitigen (EP 0 048 893, DE 29 44 457) oder nachgelagerten Umsetzung des restlichen Isobutens zu tert.-Butylethern durchgeführt.

In US 4,797,133 wird unter anderem ein Verfahren beschrieben, bei dem in einer ersten Reaktionszone Isobuten z. B. durch Reaktion zu tert.-Butylalkohol (TBA) vom Ausgangskohlenwasserstoffgemisch abgetrennt wird und der verbleibende Rest in einer Etherifizierungsstufe umgesetzt wird.

In DE 103 02 457 wird ein Verfahren zur Herstellung von Butenoligomeren und tert.-Butylethern aus Isobuten-haltigen C₄-Strömen beschrieben, bei dem das Isobuten aus einem weitgehend Butadien-freien C₄-Kohlenwasserstoffstrom mit nur geringen Verlusten an linearen Butenen vollständig entfernt werden kann. Bei diesem Verfahren wird ein Teil des Isobutens in einem ersten Reaktionsschritt an sauren Katalysatoren oligomerisiert und in einem zweiten Reaktionsschritt wird das restliche Isobuten durch Umsetzung mit Alkohol zu einem tert.-Butylether in einer Reaktivdestillationskolonne entfernt.

In DE 25 21 964 wird ein zweistufiges Verfahren zur Herstellung von Alkyl-tert.-Butylethern beschrieben, bei dem in einer ersten Stufe Isobuten mit Alkohol umgesetzt wird, der entstandene Ether abgetrennt wird und der verbleibende Rest zur Umsetzung des verbliebenen Isobutens in eine zweite Reaktionsstufe geführt wird.

Alle im Stand der Technik angeführten Verfahren mit einer partiellen Umsetzung des Isobutens weisen den Nachteil auf, dass nach Abtrennung eines Teils des Isobutens die Umsetzung des restlichen Anteils aus der Gesamtmenge der C₄-Kohlenwasserstoffe heraus erfolgt. Dies hat mindestens zwei Nachteile: zum einen die großen Mengen, die in den zweiten Reaktionsschritt gefahren werden müssen, zum anderen die relativ niedrige Konzentration an Isobuten in der Mischung. Beide Aspekte zwingen in der Regel zu einer unerwünscht großen Dimensionierung des Apparate-Equipments und verursachen meistens zudem einen erhöhten Energieverbrauch. Es bestand daher die Aufgabe, ein verbessertes Verfahren bereitzustellen, welches einen oder mehrere der genannten Nachteile nicht aufweist.

### Beschreibung der Erfindung

Überraschenderweise wurde nun gefunden, dass 1-Buten aus einer technischen Mischung von C₄-Kohlenwasserstoffen, die mindestens 1-Buten, Isobuten, n-Butan und 2-Butene enthalten, mit nur geringen Verlusten an linearen Butenen erhalten werden kann, indem ein Teil des Isobutens in einem ersten Reaktionsschritt a) zu höher als 30 °C siedenden Produkten umgesetzt wird, nicht umgesetzte C₄-Kohlenwasserstoffe aus dem Austrag von a) in Stufe b) abgetrennt werden, diese Kohlenwasserstoffe destillativ in Stufe c) aufgetrennt werden in eine mindestens 1-Buten und Isobuten aufweisende Fraktion und eine nahezu Isobuten freie, mindestens 2-Butene und n-Butan aufweisende Fraktion, das in der Isobuten enthaltende Fraktion enthaltene Isobuten mit einem Alkohol in Gegenwart eines sauren Katalysators zu Alky-tert.-Butylethern in Stufe d) umgesetzt wird, nicht umgesetzte C₄-Kohlenwasserstoffe aus dem Austrag der Stufe d) in Stufe e) abgetrennt werden und 1-Buten in Stufe f) destillativ aus dem Austrag der Stufe e) abgetrennt wird.

Gegenstand der vorliegenden Erfindung ist deshalb ein Verfahren zur Herstellung von 1-Buten aus technischen Mischungen von C₄-Kohlenwasserstoffen I, die mindestens 1-Buten, Isobuten, n-Butan und 2-Butene enthalten, welches gekennzeichnet ist durch die Verfahrensschritte
a) Umsetzung von Teilen des in der technischen Mischung enthaltenen Isobutens zu bei Normalbedingungen höher als 30 °C siedenden Produkten II,
b) Abtrennung der nicht umgesetzten C₄-Kohlenwasserstoffe III aus dem Austrag der Stufe a) durch thermische Trennverfahren,
c) destillative Trennung der C₄-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) Umsetzung des in Fraktion IV enthaltenen Isobutens mit einem Alkohol VI in Gegenwart saurer Katalysatoren zu tert.-Butylethern VII,
e) Abtrennung der nicht umgesetzten C₄-Kohlenwasserstoffe VIII aus dem Austrag von Stufe d) und
f) destillative Abtrennung des 1-Butens aus der in e) erhaltenen C₄-Kohlenwasserstoffe VIII.

Der besondere Vorteil des erfindungsgemäßen Verfahrens besteht darin, dass durch die zusätzliche Abtrennung der 2-Butene und n-Butane aus dem Reaktionsgemisch in Schritt c) ein geringer Volumenstrom durch den Reaktionsschritt d) gefahren werden muss, weshalb der oder die Reaktoren im Verfahrensschritt d) relativ klein ausgeführt sein können oder bei gleicher Größe im Vergleich zu herkömmlichen Verfahren höhere Umsätze erzielt werden können. Der weitere Vorteil der Abtrennung der 2-Butene und n-Butane in Schritt c) besteht darin, dass die Eingangskonzentration an Isobuten im Schritt d) entsprechend höher ist, was die Umsetzung des Isobutens mit Alkohol im Verfahrensschritt d) vereinfacht.

Im Vergleich zu klassischen Verfahrensweisen ergibt sich zudem durch den verringerten Volumenstrom in den Stufen d) bis f) ein deutlich geringerer Energieverbrauch des Gesamtprozesses, z. B. in Form des Wärmeträgers Dampf.

Besonders vorteilhaft kann das erfindungsgemäße Verfahren auch bei Umstellung von bestehenden Anlagen zur Herstellung von MTBE und 1-Buten auf Ethyl-tert.-butylether (ETBE) und 1-Buten eingesetzt werden. Aufgrund der ungünstigeren Gleichgewichtslage bei der Bildung von ETBE aus Isobuten und Ethanol im Vergleich zur Bildung von MTBE aus Isobuten und Methanol kommt es bei einem einfachen Wechsel des Alkohols zu einer Verringerung des Isobuten-Umsatzes. Die sich daraus ergebenden Kapazitätseinbußen können vorteilhaft über Umstellung auf das erfindungsgemäße Verfahren mindestens kompensiert werden.

Das erfindungsgemäße Verfahren zur Herstellung von 1-Buten aus technischen Mischungen von C₄-Kohlenwasserstoffen I, die mindestens 1-Buten, Isobuten, n-Butan und 2-Butene enthalten, zeichnet sich dadurch aus, dass es die Verfahrensschritte
a) Umsetzung von Teilen des in der technischen Mischung enthaltenen Isobutens zu bei Normaldruck höher als 30 °C siedenden Produkten II,
b) Abtrennung der nicht umgesetzten C₄-Kohlenwasserstoffe III aus dem Austrag der Stufe a) durch thermische Trennverfahren,
c) destillative Trennung der C₄-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) Umsetzung des in Fraktion IV enthaltenen Isobutens mit einem Alkohol VI in Gegenwart saurer Katalysatoren zu tert.-Butylethern VII,
e) Abtrennung der nicht umgesetzten C₄-Kohlenwasserstoffe VIII aus dem Austrag von Stufe d) und
f) destillative Abtrennung des 1-Butens aus den in e) erhaltenen C₄-Kohlenwasserstoffen VIII, aufweist.

### Verfahrensschritt a)

Die in Schritt a) durch Umsetzung von Isobuten erhaltenen Produkte II können z. B. durch Umsetzung mit Wasser, Alkoholen oder Formaldehyd oder durch Oligomerisierung des Isobutens hergestellt werden. Damit eine Abtrennung der Produkte von den verbleibenden nicht umgesetzten Kohlenwasserstoffen einfach, z. B. durch Destillation möglich ist, müssen die Produkte eine Siedetemperatur bei Normaldruck (101 325 Pa) von größer 30 °C aufweisen. Solche Produkte sind z. B. tert-Butyl-methylether (MTBE), tert-Butyl-ethylether (ETBE), tert-Butanol (TBA), 3-Methyl-3-buten-1-ol (MBOL), 4,4-Dimethyl-1,3-dioxan oder Diisobuten. Vorzugsweise weisen die Produkte eine Siedetemperatur bei Normaldruck von größer 45 °C und bevorzugt größer 50 °C auf. Vorzugsweise werden die Reaktionen in Schritt a) so durchgeführt, dass der Umsatz des Isobutens im Verfahrensschritt a) von größer 30 %, vorzugsweise größer 50 %, bevorzugt größer 70 % und besonders bevorzugt größer 80 % beträgt. Bei der Umsetzung von Isobuten mit Wasser oder Alkohol beträgt der Umsatz vorzugsweise größer 75 %. Die Höhe des Umsatzes an Isobuten kann z. B. durch die Anzahl der in Schritt a) verwendeten Reaktoren oder durch Wahl von geeigneten Reaktionsbedingungen, die der Fachmann leicht durch einfache Vorversuche ermitteln kann, gesteuert werden.

### Verfahrensschritt b)

Die Abtrennung der nicht umgesetzten C₄-Kohlenwasserstoffe III aus dem Austrag des Schrittes a) in Schritt b) erfolgt durch thermische Trennverfahren, wobei im Rahmen der vorliegenden Erfindung unter thermischen Trennverfahren sowohl z. B. Destillationen und Fraktionierungen als auch Extraktionen verstanden werden sollen. Beinhaltet der Verfahrensschritt a) eine Reaktivdestillation, so kann der Verfahrensschritt b) zumindest teilweise bereits bei der Durchführung der Reaktivdestillation stattfinden und ein separater Schritt b) kann gegebenenfalls entfallen.

### Verfahrensschritt c)

Nach der Abtrennung der Produkte und der gegebenenfalls vorhandenen, nicht umgesetzten Verbindungen, die als Reaktionspartner in Schritt a) zugefügt wurden, in Schritt b) wird der erhaltene Kohlenwasserstoffstrom in Schritt c) destillativ getrennt. Die destillative Trennung wird so durchgeführt, dass eine mindestens 1-Buten und Isobuten enthaltende Fraktion IV und eine nahezu Isobuten-freie, vorzugsweise weniger als 5 Massen-%, bevorzugt weniger als 1 Massen-% und besonders bevorzugt weniger als 0,1 Massen-% Isobuten aufweisende, mindestens 2-Butene und n-Butan enthaltende Fraktion V, erhalten wird. Die Fraktion V enthält mindestens 95 Massen-%, vorzugsweise mindestens 99 Massen-%, besonders bevorzugt mindestens 99,8 Massen-% der ursprünglich im als Produkt des Schrittes c) erhaltenen Kohlenwasserstoffstroms enthaltenen 2-Butene. Bevorzugt weist die Fraktion IV weniger als 1 Massen-%, besonders bevorzugt weniger als 0,2 Massen-% an n-Butan auf. Die destillative Trennung kann in für die Auftrennung solcher Kohlenwasserstoffgemische üblicherweise eingesetzten Apparaturen durchgeführt werden. Solche Apparaturen können z. B. Destillations- oder Fraktionierungskolonnen sein.

Bevorzugt wird die Trennung in einer Superfraktionierkolonne durchgeführt. Der Zulauf zu dieser Kolonne erfolgt vorzugsweise in der unteren Hälfte, bevorzugt im unteren Drittel der Kolonne. Wegen der engen Siedelage des zu trennenden Gemisches wird die Kolonne mit vorzugsweise mehr als 100, bevorzugt mehr als 125, besonders bevorzugt mit 150 oder mehr theoretischen Trennstufen und ganz besonders bevorzugt mit 150 bis 200 theoretischen Trennstufen ausgeführt. Das Rücklaufverhältnis (Rücklaufmenge zu Destillatabnahme) beträgt, in Abhängigkeit von der realisierten Stufenzahl und vom Betriebsdruck, vorzugsweise kleiner-gleich 20, bevorzugt kleiner 14, besonders bevorzugt kleiner 11. Die Kondensation kann gegen Kühlwasser oder Luft durchgeführt werden. Der Destillatbehälter wird vorzugsweise als Flüssigkeit-Flüssigkeit-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Die Trennung gemäß Verfahrensschritt c) wird vorzugsweise bei einem Druck von 4 bis 10 bar_{absolut} (bara), bevorzugt bei einem Druck von 5 bis 7 bara durchgeführt. Die Temperatur bei welcher die Trennung durchgeführt wird beträgt vorzugsweise von 35 bis 65 °C, bevorzugt 40 bis 50 °C.

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonnen, die außerhalb des erfindungsgemäßen Verfahren am Anlagenstandort vorhanden ist, mit der erfindungsgemäßen Kolonne des Verfahrensschrittes c) verschaltet werden. Besonders bevorzugt ist die zweite Kolonne die C₄-Trennkolonne aus Verfahrensschritt f).

Die in Schritt c) erhaltene Fraktion V kann als Alkylierungsmittel verwendet werden. Insbesondere eignet sie sich zur Herstellung von n-Buten-Oligomeren, insbesondere von Di-n-Buten oder Tributen, z. B. nach dem OCTOL-Verfahren der OXENO Olefinchemie GmbH, wie es in DE 196 29 906 oder EP 0 395 857 beschrieben wird.

### Verfahrensschritt d)

Die aus Schritt c) erhaltene Isobuten-haltige Fraktion IV wird im erfindungsgemäßen Verfahren in einem weiteren Reaktionsschritt (Schritt d) umgesetzt, bei der das restliche Isobuten durch Anlagerung von Alkohol zum entsprechenden tertiären Ether umgesetzt wird.

Die Veretherung des Isobutens wird als sauer katalysierte Reaktion durchgeführt. Als Alkohole können primäre, sekundäre, ein- oder mehrwertige Alkohole vorzugsweise mit 1 bis 5 C-Atomen, besonders bevorzugt Methanol oder Ethanol eingesetzt werden. Als Alkohol kann hochreiner Alkohol, reiner Alkohol oder Alkohol verwendet werden, der geringe Mengen an Verunreinigungen aufweist. Bevorzugt liegt die Reinheit des eingesetzten Alkohols, angegeben in Massen-% an Alkohol, über 90%, besonders bevorzugt über 95 %, ganz besonders bevorzugt über 99 %. Der Gehalt an Wasser liegt bevorzugt unter 3 Massen-%, besonders bevorzugt unter 1 Massen-%, ganz besonders bevorzugt unter 0,3 Massen-%.

Für die Umsetzung von Isobuten mit Alkoholen, insbesondere mit Methanol zu Methyl-tert.-butylether wurden diverse Verfahrensvarianten entwickelt (vergleich: Ullmann's Encyclopedia of Industrial Chemistry, Online Version, 2004, Wiley & Sons, Stichwort Methyl-tert.-Butylether, und dort zitierte Literatur; Obenaus, Fritz; Droste, Wilhelm, Erdoel & Kohle, Erdgas, Petrochemie (1980), 33(6), 271-275; DE 26 29 769; DE 28 53 769). Prinzipiell sind alle bekannten Verfahren zur Umsetzung des Isobutens mit Alkoholen geeignet als Verfahrensschritt d) im Rahmen der vorliegenden Erfindung eingesetzt zu werden.

Bevorzugt werden Verfahren, bei denen die Umsetzung in flüssiger Phase an einem sauren Ionenaustauscherharz erfolgt, eingesetzt. Als Reaktoren, in denen der Alkohol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Sie können mit oder ohne partieller Rückführung, wobei ggf. der Rückführungsstrom gekühlt werden kann, betrieben werden.

Die Reaktoren können bei Temperaturen von 10 bis 160 °C, bevorzugt bei Temperaturen von 30 bis 110 °C, betrieben werden. Der Druck beträgt vorzugsweise 5 bis 50 bara, bevorzugt 10 bis 20 bara. Da das thermodynamische Gleichgewicht zwischen Alkohol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben.

Das molare Verhältnis von Alkohol zu Isobuten im Zulauf zum Verfahrenschritt d) liegt vorzugsweise im Bereich von 10 zu 1 bis 1 zu 1, besonders bevorzugt von 5 zu 1 bis 1,1 zu 1 und ganz besonders bevorzugt im Bereich von 3 zu 1 bis 1,2 zu 1. Als Katalysator wird sowohl in den Festbettstufen als auch in einer gegebenenfalls vorhandenen Reaktivdestillationskolonne ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen keine sauren Stoffe an das Produktgemisch abgeben, weil dies zu Ausbeuteverluste führen kann.

Die Aktivität der Katalysatoren wird vorzugsweise so gewählt, dass sie unter Reaktionsbedingungen die Addition von Alkohol an Isobuten katalysieren, jedoch kaum die Addition an lineare Butene. Weiterhin sollten die Katalysatoren die Oligomerisierung von linearen Butenen und die Dialkyletherbildung aus zwei Molekülen eingesetzten Alkohols möglichst nicht oder nur wenig katalysieren. Im Hinblick auf eine hohe Ausbeute an 1-Buten sollte die Aktivität für die Isomerisierung von 1-Buten zu 2-Buten vorzugsweise gering sein.

Als feste Katalysatoren können beispielsweise Zeolithe, säureaktivierte Bentonite und/oder Tonerden, sulfonierte Zirkoniumoxide, Montmorillonite oder saure Ionenaustauscherharze verwendet werden.

Eine im erfindungsgemäßen Verfahren bevorzugte Gruppe von sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, bevorzugt von 0,7 bis 2,0 eq/l, insbesondere von 1,1 bis 2,0 eq/l bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

In einer bevorzugten Ausführungsform wird die Addition des Alkohols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt d) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus der Isobuten-haltigen Fraktion IV und dem Alkohol VI ein Reaktionsgemisch hergestellt, das hinsichtlich seiner Isobuten-, Alkohol- und tert.-Butylether-Konzentration in der Nähe des thermodynamischen Gleichgewichts liegt. Der Umsatz des Isobutens beträgt dabei bevorzugt größer 90 %. Dieses Gemisch wird in der nächsten/letzten Reaktionsstufe in die Reaktivdestillationskolonne eingespeist, wo ein weiterer Teil des Isobutens zum Ether umgesetzt wird.

Im Reaktionsteil der Reaktivdestillationskolonne können die gleichen Katalysatoren, wie die oben für die einfache Ausführungsform der Verfahrensstufe, ohne die Verwendung einer Reaktivdestillation, beschrieben, eingesetzt werden.

In der Reaktivdestillationskolonne kann der Katalysator entweder in der Packung integriert sein, beispielsweise KataMax^{®} (wie in EP 0 428 265 beschrieben), KataPak^{®} (wie in EP 0 396 650 oder DE 298 07 007.3 beschrieben) oder auf Formkörpern aufpolymerisiert (wie in US 5 244 929 beschrieben) sein.

Die Umsetzung des Isobutens mit Alkohol zum entsprechenden tertiären Butylether erfolgt in der Reaktivdestillation vorzugsweise im Temperaturbereich von 10 bis 140 °C, bevorzugt bei 40 bis 90 °C, besonders bevorzugt bei 60 bis 80 °C (Bereich der Kolonne, in der sich der Katalysator befindet. Die Sumpftemperatur der Kolonne kann deutlich höher liegen).

Insbesondere wird das Isobuten durch Umsetzung mit Methanol zu MTBE oder mit Ethanol zu ETBE entfernt. Dabei wird insbesondere derart verfahren, wie es in DE 101 02 082 für die Reaktion von Methanol mit Isobuten zu MTBE beschrieben ist. Das Isobuten aufweisende C₄-Kohlenwasserstoffgemisch wird zusammen mit Alkohol (Methanol oder Ethanol) in den/die Vorreaktor(en) eingespeist. Dabei wird der Alkohol bevorzugt im Überschuss eingesetzt. In den Vorreaktoren entsteht ein Gemisch, in dem Isobuten, Alkohol (Methanol, Ethanol) und korrespondierender Alkyl-tert.-Butylether (ATBE) im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Alkohol (Methanol, Ethanol) enthalten sein, als für die vollständige Umsetzung des noch vorhandenen Isobutens gebraucht wird. Der Alkoholüberschuss sollte jedoch derart bemessen werden, dass eine ausreichende Alkoholmenge für das sich bildende Azeotrop aus Alkohol (Methanol, Ethanol) und C₄-Kohlenwasserstoffen vorhanden ist.

Optional kann, beispielsweise wenn der Alkoholgehalt im Kolonnenzulauf unter dem maximal zulässigen Wert liegt, zusätzlicher Alkohol zum Kolonnenzulauf zugemischt werden. Darüber hinaus kann am Kopf der Reaktivdestillationskolonne oder an anderen Stellen, beispielsweise direkt oberhalb oder in einem Flüssigkeitsverteiler der katalytischen Packungen, über eine gesonderte Einrichtung eine Alkohol-Einspeisung erfolgen.

Bevorzugt weist die Reaktivdestillationskolonne oberhalb der Katalysatorpackung einen Bereich rein destillativer Trennung auf. Bevorzugt weist die Zone oberhalb der Katalysatorpackung 5 bis 20, insbesondere 10 bis 15 Trennstufen auf. Die Trennzone unterhalb des Katalysators umfasst 12 bis 36, insbesondere 20 bis 30 Trennstufen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 75 bis 99 %, vorzugsweise von 85 bis 98 % und besonders bevorzugt von 95 bis 97 % bezogen auf den Isobutengehalt im Zulauf zur Reaktivdestillation erreicht wird.

Der Zulauf zur Reaktivdestillationskolonne kann oberhalb oder unterhalb, vorzugsweise unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Die Reaktivdestillationskolonne wird bei Drücken, gemessen am Kolonnenkopf, von 3 bara bis 25 bara, bevorzugt 5 bara bis 15 bara betrieben, insbesondere von 7 bara bis 10 bara. Die hydraulische Belastung in der katalytischen Packung der Kolonne beträgt bevorzugt 10 % bis 110 %, vorzugsweise 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden. (Vauck/Müller, "Grundoperationen chemischer Verfahrenstechnik", S. 626, VEB Deutscher Verlag für Grundstoffindustrie.)

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen von 0,2 bis 4 betrieben, insbesondere mit solchen, die von 0,4 bis 2, bevorzugt von 0,5 bis 1 betragen.

Werden für die Veretherung andere Alkohole als Methanol oder Ethanol eingesetzt, so ändern sich die Parameter der Reaktivdestillation entsprechend.

Wird in der Stufe d) als letzter Schritt eine Reaktivdestillationskolonne eingesetzt, so kann in dieser zumindest teilweise der Schritt d) sowie der Schritt e), nämlich die Abtrennung des ATBE von den nicht umgesetzten Kohlenwasserstoffen stattfinden. Auf einen weiteren Schritt e) kann dann gegebenenfalls verzichtet werden.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht.

Es ist im erfindungsgemäßen Verfahren auch möglich, diese Bereiche räumlich zu trennen, ohne auf die Vorteile einer Reaktivdestillation zu verzichten. In einer Verfahrensvariante wird die Reaktivdestillationskolonne als Destillationskolonne mit einem oder mehreren außenliegenden Reaktor(en), die den Katalysator enthält/enthalten und im Nebenstrom betrieben wird/werden, ausgeführt.

### Verfahrensschritt e)

Wird in Verfahrensschritt d) keine Reaktivdestillationskolonne zur Veretherung und gleichzeitigen Trennung eingesetzt, so muss im erfindungsgemäßen Verfahren ein eigenständiger Schritt e) vorgesehen werden, in welchem das Produkt aus dem Verfahrensschritt d) getrennt wird in den Alkyl-tert.-butylether sowie die nicht umgesetzten Kohlenwasserstoffe. Die Trennung kann z. B. dadurch erfolgen, dass der Austrag aus dem Reaktor des Verfahrensschrittes d) in eine Destillationskolonne eingespeist wird. Die Kolonne kann mit einem Sumpfverdampfer und einem Kondensator für das Kopfprodukt ausgerüstet sein. Als Sumpfprodukt der Destillationskolonne wird ATBE und ggf. überschüssiger Alkohol erhalten. Das Kopfprodukt kann teilweise als Rücklauf in die Kolonne zurückgefahren werden. Der andere Teil kann dem Verfahrensschritt f) zugeführt werden.

Die Kolonne weist vorzugsweise mehr als 20, bevorzugt mehr als 25, besonders bevorzugt mehr als 30 theoretischen Trennstufen auf. Das Rücklaufverhältnis ist, in Abhängigkeit von der realisierten Stufenzahl, vorzugsweise kleiner-gleich 2, besonders bevorzugt kleiner 1. Die Kondensation kann gegen Kühlwasser oder Luft durchgeführt werden. Zur Beheizung des Verdampfers der Kolonne kann z. B. Dampf eingesetzt werden. Es kann vorteilhaft sein, den Zulaufstrom zur Kolonne zumindest teilweise vorverdampft in die Kolonne zu leiten oder direkt in die Kolonne zu flashen. Bevorzugt wird dafür dem Zulaufstrom in einem externen Wärmeübertrager Wärme, z. B. durch Nutzung von Abwärme, zugeführt. Zum Erzielen einer teilweisen Verdampfung ist ein Kettle-Verdampfer die bevorzugte Ausführungsform des Wärmeübertragers. Es kann außerdem vorteilhaft sein, wenn ein mit Prozess- oder Abwärme auf niedrigerem Temperaturniveau beheizter Zwischenverdampfer im unteren Teil der Kolonne eingesetzt wird.

Weist das Kopfprodukt von Verfahrensschritt e) unabhängig davon, ob der Schritt in einer Destillations- oder Reaktivdestillationskolonne durchgeführt wurde, noch Restmengen an Alkohol in den C₄-Kohlenwasserstoffen auf, so kann es vorteilhaft sein, diese in mindestens einem zusätzlichen Verfahrensschritt zu entfernen. Verfahren zur Abtrennung von Alkoholen, insbesondere von Methanol oder Ethanol, gehören zum Stand der Technik. Die Abtrennung kann beispielsweise über Adsorption an Molsieben, Membranverfahren, Schleppmitteldestillation oder Extraktion erfolgen. Besonders elegant kann die Abtrennung in einem Extraktionsschritt durch Extraktion des Alkohols mit Wasser erfolgen. Diese Wäsche erfolgt nach den bekannten Standardverfahren der Technik, beispielsweise in einer Extraktionskolonne oder in einer Kaskade von Mixern und Trennbehältern. Gegenüber den anderen Verfahren weist sie verschiedene Vorteile auf, beispielsweise geringes Investment und niedrige Betriebskosten.

Bei Einsatz von Methanol oder Ethanol als Alkohol werden Restmengen des Alkohols in den C₄-Kohlenwasserstoffen bevorzugt in einer Extraktionskolonne mit Wasser entfernt. Der Restgehalt an Alkohol in den C₄-Kohlenwasserstoffen wird dabei bevorzugt auf unter 0,2 %, besonders bevorzugt auf unter 500 ppm, ganz besonders bevorzugt auf unter 50 ppm abgesenkt. Die Extraktionskolonne weist bevorzugt 25 bis 2, besonders bevorzugt 15 bis 5 theoretische Trennstufen auf und wird bevorzugt bei Temperaturen von 10 bis 90 °C und Drücken von mindestens einem bar über dem Dampfdruck der C₄-Kohlenwasserstoffe betrieben.

Das mit Alkohol beladene Waschwasser aus der Extraktion wird bevorzugt in einer separaten Einheit aufgearbeitet und zumindest teilweise in die Extraktion zurückgeführt. Die Aufarbeitung kann beispielsweise durch eine Destillation erfolgen, bei der eine praktisch alkoholfreie Wasserfraktion im Sumpf und eine alkoholreiche Fraktion als Kopfprodukt erhalten werden.

Bevorzugt wird das Kopfprodukt der Destillationskolonne oder Reaktivdestillationskolonne in eine Extraktionskolonne überführt, in die im Gegenstrom ein Extraktionsmittel, wie z. B. Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion der Strom aus in Stufe d) und gegebenenfalls e) nicht umgesetzten Kohlenwasserstoffen VIII erhalten, der Schritt f) zugeführt wird.

Der in der Reaktivdestillation bzw. Destillation der Schritte d) bzw. e) als Sumpfprodukt anfallende Alkyl-tert.-butylether, der ggf. noch Restmengen an Alkohol enthält, kann für verschiedene Zwecke genutzt werden. Neben dem Einsatz als Komponente für Ottokraftstoffe findet er beispielsweise Verwendung als Lösungsmittel. Durch Rückspaltung der tert.-Butylether ist Isobuten hoher Reinheit erhältlich.

Der bei Verwendung von Methanol anfallende MTBE wird beispielsweise, neben der Verwendung als Komponente im Ottokraftstoff, als Lösungsmittel genutzt. Zur Gewinnung von MTBE hoher Reinheit, welcher bevorzugt als Lösungsmittel eingesetzt wird, kann der im Prozess anfallende MTBE destillativ weiter aufgereinigt werden. Dabei wird der Gehalt an in geringer Menge enthaltenen Verunreinigungen (beispielsweise Methyl-sec.-butylether, C₈-KW, TBA, Alkohole) reduziert.

Die Rückspaltung von MTBE zur Gewinnung von Isobuten ist beispielsweise in DE 100 20 943 beschrieben. Die dabei erhaltene Reinheit des Isobutens ist unter anderem abhängig vom Anteil an Methyl-sec.-butylether im MTBE. Je nach Anforderungen wird daher ein unterschiedlich intensiv vorgereinigtes MTBE für die Rückspaltung eingesetzt.

In einer bevorzugten Ausführungsform des Verfahrens wird die Umsetzung in Schritt d) mit einem stöchiometrischen Überschuss an Alkohol betrieben und das in der Reaktivdestillation bzw. Destillation der Schritte d) bzw. e) anfallende Sumpfprodukt, welches Alkyl-tert.-butylether und Restmengen Alkohol enthält, ganz oder teilweise in den Schritt a) und/oder b) zurückgeführt. Das Verhältnis Alkohol zu Isobuten, welches in den Schritt d) geführt wird, liegt dabei bei 1,1 bis 10 mol/mol, bevorzugt bei 1,2 bis 5 mol/mol. Bevorzugt wird bei dieser Variante in Schritt d) die Umsetzung nur in Festbettreaktoren durchgeführt und in Schritt e) eine destillative Trennung in einer Destillationskolonne durchgeführt. Die nicht umgesetzten C₄-Kohlenwasserstoffe VIII werden als Kopfprodukt erhalten und das anfallende Sumpfprodukt, welches mindestens tert.-Butylether und Restmengen an Alkohol enthält, wird ganz oder teilweise in den Schritt a) und/oder b) zurückgeführt.

### Verfahrensschritt f)

Aus dem aus der Reaktivdestillation bzw. Destillation gemäß Schritt e) erhaltenen und ggf. von Alkohol befreiten C₄-Kohlenwasserstoffgemisch VIII aus nicht umgesetzten Kohlenwasserstoffen, die im wesentlichen 1-Buten, Isobutan und Leichtsieder aufweist, wird destillativ 1-Buten abgetrennt. Die Abtrennung des 1-Butens erfolgt durch Destillation des Gemisches VIII in einer oder mehreren Destillationskolonnen.

In einer bevorzugten Ausführungsform erfolgt die Abtrennung des 1-Butens in einer Destillationskolonne, in der als Sumpfprodukt sehr reines 1-Buten erhalten wird. Als Kopfprodukt wird eine Isobutan-reiche Fraktion erhalten, die zudem Leichtsieder (beispielsweise C₃-Kohlenwasserstoffe) enthält.

Bevorzugt wird die Trennung in einer Superfraktionierkolonne durchgeführt. Der Zulauf zu dieser Kolonne erfolgt vorzugsweise in die obere Hälfte, bevorzugt in die untere Hälfte der oberen Hälfte der Kolonne. Wegen der engen Siedelage des zu trennenden Gemisches wird die Kolonne mit vorzugsweise mehr als 100, bevorzugt mehr als 125, besonders bevorzugt mehr als 150 und ganz besonders bevorzugt von 150 bis 200 theoretischen Trennstufen ausgeführt. Das Rücklaufverhältnis (Rücklaufmenge zu Destillatabnahme) ist, in Abhängigkeit von der realisierten Stufenzahl und vom Betriebsdruck, vorzugsweise kleiner-gleich 100, bevorzugt kleiner 70, besonders bevorzugt kleiner 60. Ganz besonders bevorzugt beträgt das Rücklaufverhältnis von 30 bis 60. Die Kondensation kann gegen Kühlwasser oder Luft durchgeführt werden. Der Destillatbehälter wird vorzugsweise als flüssig-flüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonnen, die außerhalb des erfindungsgemäßen Verfahren am Anlagenstandort vorhanden ist, mit der erfindungsgemäßen Kolonne des Verfahrensschrittes f) verschaltet werden. Besonders bevorzugt ist die zweite Kolonne die C₄-Trennkolonne aus Verfahrensschritt c). Dabei wird eine der Kolonnen bei so hohem Druck gefahren, dass ihre Kondensationstemperatur zur Beheizung der anderen Kolonne ausreicht.

In einer weiteren bevorzugten Ausführungsform des Verfahrensschritts f) werden in einer ersten Destillationskolonne Leichtsieder als Kopfprodukt abgetrennt; im Sumpf der Kolonne wird eine Mischung erhalten, die hauptsächlich 1-Buten und Isobutan enthält. In einer zweiten Kolonne wird diese Sumpfmischung in 1-Buten, welches als Sumpfprodukt anfällt, und eine Isobutan-reiche Fraktion (Kopfprodukt), getrennt.

Mit dem erfindungsgemäßen Verfahren hergestelltes, reines 1-Buten enthält vorzugsweise weniger als 5000 wppm (Massen-ppm), bevorzugt weniger als 2000 wppm und besonders bevorzugt weniger als 1500 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd. Eine weitere Verwendung des erfindungsgemäß hergestellten nahezu Isobuten-freien 1-Butens ist die Herstellung von n-Buten-Oligomeren, insbesondere nach dem Octol-Prozess.

Im Verfahrensschritt f) fallen neben dem 1-Buten üblicherweise (je nach Ausgangszusammensetzung der C₄-Kohlenwasserstoffe) Isobutan-reiche Fraktionen an. Diese können weiter, vorzugsweise zu reinem Isobutan, aufgereinigt werden. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine. Eine Aufreinigung zu reinem Isobutan kann beispielsweise durch vollständige Hydrierung der noch enthaltenen Alkene zu Alkanen und anschließende Destillation erfolgen.

### Ausführungsform 1: Umsetzung von Isobuten mit Wasser in Schritt a)

In einer ersten bevorzugten Ausführungsform des erfindungsgemäßen Verfahren wird das in dem technischen C₄-Kohlenwasserstoffgemisch enthaltene Isobuten in Schritt a) mit Wasser zu tert-Butanol (TBA) umgesetzt. Der Schritt a) kann so durchgeführt werden, dass Verfahren zur Herstellung von TBA angewendet werden. Als bevorzugte Verfahren zur Umsetzung des Isobutens mit Wasser zu TBA werden Prozesse mit einem heterogenen Katalysator eingesetzt. Solche Verfahren werden z. B. in DE 10 2004 030 943, DE 103 30 710, EP 0 579 153, US 6,111,148 oder DE 30 25 262 beschrieben.

Die Herstellung von tertiärem Butanol (TBA) durch Umsetzung des isobutenhaltigen C₄-Kohlenwasserstoffstroms I mit Wasser erfolgt vorzugsweise an einem sauren Ionenaustauscherharz. Der Schritt a) kann in einem, zwei oder mehreren Reaktoren stattfinden. Es kann außerdem vorteilhaft sein, wenn zumindest eine Verfahrensstufe, vorzugsweise die letzte Verfahrensstufe der Herstellung von TBA in Schritt a) eine Reaktivdestillation umfasst.

Bevorzugt wird ein Verfahren zur Umsetzung von Isobuten mit Wasser angewandt, bei dem die Umsetzung in mehreren Stufen an einem sauren Ionentauscherharz erfolgt. Dabei liegt bevorzugt nur eine flüssige Phase vor. In die erste Stufe werden die C₄-Kohlenwasserstoffe, Wasser und gegebenenfalls ein Cosolvens, welches bei einer Temperatur höher als die Siedetemperatur von C₄-Kohlenwasserstoffen siedet, bevorzugt TBA, eingebracht. Durch das Cosolvens kann der Anteil an Wasser in der Mischung erhöht werden, ohne dass sich zwei flüssige Phasen ausbilden.

Da durch die Hydratisierung von Isobuten Wasser verbraucht wird, sinkt der Wassergehalt in der Reaktionsmischung. Um eine möglichst hohe Ausbeute und/oder Reaktionsgeschwindigkeit zu erhalten, kann es daher, wenn mehrere Reaktionsstufen vorhanden sind, vorteilhaft sein, vor den nachfolgenden Stufen jeweils Wasser nachzudosieren.

Erfindungsgemäß werden bevorzugt nur homogene Lösungen den Reaktoren zugeführt. Daher müssen Wasser bzw. Wasser-tert.-Butanol-Lösungen mit dem Einsatz-Kohlenwasserstoffgemisch oder einem Reaktoraustrag gemischt werden, so dass bis zum Eintritt in den ersten Reaktor bzw. einer der folgenden Reaktoren eine homogene Lösung entstanden ist. Dies kann beispielsweise unter Verwendung von Statikmischern erreicht werden. Die Einstellung der gewünschten Wasserkonzentration im Reaktorzulauf kann durch Mengenregelung der einzelnen Ströme nach Messung von deren Wassergehalten erfolgen.

Die in die einzelnen Stufen zudosierte Wassermenge wird vorzugsweise so bemessen, dass am Reaktoreingang eine einzige flüssige Phase vorliegt. Bevorzugt wird ein Wasseranteil von 30 bis 100 Massen-%, besonders bevorzugt von 70 bis 98 Massen-% der durch die Löslichkeit von Wasser im Reaktionsgemisch möglichen Wassermenge eingesetzt.

Das Verfahren gemäß der Erfindung kann in chargenweise oder kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt.

Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden. Reaktoren, bei denen ein Teil des Reaktionsgemisches zurückgeführt wird, können mit Leerrohrgeschwindigkeiten von vorzugsweise 10 bis 30 m/h betrieben werden. In den Reaktoren, die im geraden Durchgang durchströmt werden, können die Leerrohrgeschwindigkeiten vorzugsweise im Bereich von 1 bis 20 m/h liegen.

Dementsprechend beträgt die Katalysatorbelastung (LHSV) bei Reaktoren, die unter Produktrückführung betrieben werden, vorzugsweise 0,3 bis 20 h⁻¹, bevorzugt 1 bis 10 h⁻¹. Bei Reaktoren, die im geraden Durchgang durchströmt werden, liegen die Belastungen vorzugsweise im Bereich von 0,1 bis 5,0 h⁻¹, bevorzugt im Bereich von 0,4 bis 3 h⁻¹.

Das erfindungsgemäße Verfahren kann in einem, aber auch in mehreren, insbesondere 2, 3 oder 4 hintereinandergeschalteten Reaktor(en), die in Fließrichtung sinkende Temperaturen aufweisen können, durchgeführt werden.

Wird der erste Reaktor oder mehrere Reaktoren unter Produktrückführung betrieben, wird vorzugsweise ein Kreislauffaktor (Verhältnis vom im Kreis gepumpten Menge zu Frischzulauf) von 0,1 bis 10 eingestellt. Dabei beträgt der Kreislauffaktor für den ersten Reaktor bevorzugt 1 bis 4, insbesondere 2 bis 3,5.

Eine bevorzugte Verfahrensvariante besteht darin, den ersten Reaktor unter Produktrückführung und die weiteren Reaktoren im geraden Durchgang zu betreiben. Die Anzahl der verwendeten Reaktoren beträgt abhängig vom angestrebten Umsatz vorzugsweise 2 bis 10, insbesondere 2 bis 4.

Jeder Reaktor kann adiabatisch oder praktisch isotherm, d. h., mit einem Temperaturanstieg unter 10 °C betrieben werden. Ein zu hoher Temperaturanstieg ist wegen der ungünstigen Gleichgewichtsbeeinflussung (Rückspaltung) und möglicher Nebenproduktbildung zu vermeiden.

Die Temperaturen, bei denen der Schritt a) des erfindungsgemäßen Verfahrens durchgeführt wird, betragen vorzugsweise von 30 bis 120 °C. Bei tieferen Temperaturen ist die Reaktionsgeschwindigkeit zu gering und bei höheren treten vermehrt Nebenreaktionen, wie beispielsweise die Oligomerisierung der Olefine auf. Vorzugsweise werden die Reaktoren im Temperaturbereich 35 bis 70 °C betrieben. Die Temperaturen in verschiedenen Reaktoren können gleich oder verschieden innerhalb des angegebenen Bereichs sein. Eine Verfahrensvariante besteht darin, die Temperatur in Flussrichtung von Reaktor zu Reaktor zu senken. Da mit sinkender Temperatur die Gleichgewichtslage günstiger wird, kann somit ein höherer Umsatz erreicht werden. Allerdings ist es nicht sinnvoll, die Temperatur unter 35 °C zu senken, da dann die Reaktion für ein technisches Verfahren zu langsam wird.

Die erfindungsgemäße Umsetzung gemäß dieser Ausführungsform des Verfahrensschrittes a) wird vorzugsweise bei einem Druck gleich oder über dem Dampfdruck des Einsatz-Kohlenwasserstoffgemisches bei der jeweiligen Reaktionstemperatur, vorzugsweise bei einem Druck unter 40 bar, durchgeführt. Um in den Reaktoren Verdampfungsprobleme zu vermeiden, sollte der Druck vorzugsweise mindestens 2 bis 4 bar höher als der Dampfdruck des Reaktionsgemisches sein.

Der Gesamtumsatz an Isobuten hängt von der Art und Menge des verwendeten Katalysators, den eingestellten Reaktionsbedingungen und Anzahl der Reaktionsstufen ab. Aus wirtschaftlichen Gründen wird der Isobuten-Umsatz im Bereich von 50 bis 95 %, vorzugsweise zwischen 70 und 90 % gehalten.

Der Verfahrensschritt b) des erfindungsgemäßen Verfahrens, die Abtrennung der nicht umgesetzten C₄-Kohlenwasserstoffe III von den Produkten II, erfolgt bevorzugt destillativ, insbesondere durch Zuführung des den letzten Reaktor verlassenden Reaktionsgemisches in eine Destillationskolonne, die bei oder unter dem Druck des letzten Reaktors, jedoch mindestens bei einem Druck von über 1 bar arbeitet. Bei der Destillation fallen als Kopfprodukt die nicht umgesetzten Kohlenwasserstoffe III an. Da die C₄-Kohlenwasserstoffe Azeotrope mit Wasser bilden, fällt bei der Kondensation der Brüden am Kolonnenkopf zudem eine polare, wässrige Phase neben den C₄-Kohlenwasserstoffen an. Diese wird bevorzugt abgetrennt. Dies erfolgt entsprechend den Standardverfahren der Technik, beispielsweise in einem Kopfdekanter. Bevorzugt erfolgt deshalb die Abtrennung der C₄-Kohlenwasserstoffe III vom TBA/Wasser-Gemisch zusammen mit etwas Wasser. Die Kohlenwasserstoffe III können vor oder nach der Wasserabtrennung, vorzugsweise nach der Wasserabtrennung in Verfahrensschritt c) des erfindungsgemäßen Verfahrens eingesetzt werden.

Als Sumpfprodukt wird eine wässrige tert.-Butanol-Lösung gewonnen. Ein Teil des Sumpfprodukts wird bevorzugt in den Verfahrensschritt a), vorzugsweise in den ersten Reaktor des Verfahrensschrittes a), zurückgefahren (rezykliert). Bevorzugt werden dabei 0,1 bis 2 t, besonders bevorzugt 0,2 bis 0,5 t Sumpfprodukt pro Tonne an zugeführten C₄-Kohlenwasserstoffen rezykliert.

Das Sumpfprodukt kann als solches verwendet oder weiter aufgearbeitet werden. Typische weitere Aufreinigungen erfolgen zu tert.-Butanol (wasserfrei) und dem Azeotrop aus Wasser und tert.-Butanol. Für die Herstellung von wasserfreiem tert.-Butanol sind in der Literatur mehrere Verfahren bekannt (DE 102 41 762 und dort zitierter Stand der Technik).

Weiterhin ist es möglich einen anderen Strom, bestehend aus Wasser und tert.-Butanol, der bei der Aufarbeitung des Roh-tert.-Butanol anfällt, in den Verfahrensschritt a), vorzugsweise in den ersten Reaktor des Verfahrensschrittes a) zurückzuführen.

Wird ein Cosolvens eingesetzt, so wird dieses wegen der Siedetemperatur auch im Sumpf der Destillation anfallen. Es kann über geeignete thermische Trennverfahren aus dem Sumpfprodukt abgetrennt und vollständig oder teilweise in den Verfahrensschritt a), bevorzugt in den ersten Reaktor des Verfahrensschrittes a) zurückgeführt werden.

Als eigentlicher Katalysator wird in allen (beiden) Stufen dieser Ausführungsart des Verfahrensschrittes a) ein fester Stoff, der weder im Einsatzstoffgemisch noch im Produktgemisch löslich ist, mit sauren Zentren an seiner Oberfläche eingesetzt. Vorzugsweise wird ein wie in Verfahrenschritt d) beschriebener Katalysator als Katalysator in dieser Ausführungsart des Verfahrensschrittes a) eingesetzt. Der Katalysator kann in allen einsetzbaren Reaktoren eingesetzt werden, also z. B. sowohl in Festbettreaktoren als auch im Reaktionsteil von Reaktivdestillationskolonnen. Bevorzugt in dieser Verfahrensvariante einsetzbare saure Katalysatoren sind feste Ionenaustauscherharze mit Sulfonsäuregruppen. In der erfindungsgemäßen Ausführungsform des Schrittes a) können die Ionenaustauscherharze in ihrer H-Form eingesetzt werden. Stark saure Harze des Styrol-Divinylbenzol-Typs werden u. a. unter folgenden Handelsnamen verkauft: Lewatit SCP 118, Lewatit SCP 108, Amberlyst 15 oder Amberlyst 35, Lewatit K2621, Lewatit K2629, Lewatit K2431. Das Porenvolumen beträgt vorzugsweise 0,3 bis 0,9 ml/g, insbesondere 0,5 bis 0,9 ml/g. Die Korngröße des Harzes liegt vorzugsweise zwischen 0,3 mm und 1,5 mm, insbesondere zwischen 0,5 mm und 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Säurekapazität des Ionenaustauscher beträgt, bezogen auf die Lieferform, vorzugsweise von 0,7 bis 2,0 eq/1, insbesondere von 1,1 bis 2,0 eq/1 bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

In einer weiteren bevorzugten Variante der Ausführungsform 1 wird die letzte Stufe als Reaktivdestillation ausgeführt. Ein Verfahren zur Herstellung von TBA über das Verfahren der Reaktivdestillation ist beispielsweise in DE 102 60 991 beschrieben.

Die Einspeisung des Isobuten-haltigen C₄-Kohlenwasserstoffstrom und des Wassers bzw. des Gemisches aus C₄-Kohlenwasserstoffstrom, TBA und Wasser in die Reaktivdestillationskolonne erfolgt vorzugsweise unterhalb der Reaktionszone. Zwischen Zulauf und Reaktionszone kann sich ein destillativer Bereich befinden. Unterhalb des reaktiven Bereichs befindet sich ein rein destillativer Bereich, welcher zur Abtrennung des TBA und gegebenenfalls überschüssigen Wassers dient. Das Sumpfprodukt der Reaktivdestillation enthält überwiegend TBA und Wasser, idealerweise mit Wasserkonzentrationen, geringer als im Wasser/TBA-Azeotrop. Optional kann oberhalb des reaktiven Bereichs ein weiterer destillativer Bereich folgen, um die Isobuten-Konzentration in der Reaktionszone einzustellen. Als Kopfprodukt werden die nicht umgesetzten C₄-Kohlenwasserstoffe III erhalten. Der Verfahrensschritt b) ist bei dieser Variante somit Teil des Schritts a).

Da die C₄-Kohlenwasserstoffe Azeotrope mit Wasser bilden, fällt bei der Kondensation der Brüden am Kolonnenkopf zudem eine polare, wässrige Phase neben den C₄-Kohlenwasserstoffen III an. Diese wird bevorzugt abgetrennt und optional ganz oder teilweise in die Kolonne zurückgeführt. Die Abtrennung erfolgt entsprechend den Standardverfahren der Technik, beispielsweise in einem Kopfdekanter. Diese Verfahrensvariante hat den Vorteil, dass das Reaktionswasser weitgehend im Kreis geführt wird und das Kopfprodukt der Kolonne nur noch geringe Mengen an Wasser enthält. Optional kann ein Teil der organischen Destillatphase oder ein Teil des gesamten Destillats in die Reaktivdestillationskolonne zurückgeführt werden. Die Rückführung der wässrigen Destillatphase kann oberhalb und/oder unterhalb, die der organischen Destillatphase oberhalb der Reaktionszone erfolgen. Die auf diese Art erhaltenen C₄-Kohlenwasserstoffe III sind typischerweise wassergesättigt aber frei von heterogenen Wasseranteilen. Sie können so in Verfahrensschritt c) des erfindungsgemäßen Verfahrens eingesetzt werden.

Der Zulauf zur Reaktivdestillation enthält bevorzugt kleiner 20 Massen-%, insbesondere kleiner 15 Massen-% Isobuten, das in der Reaktivdestillationskolonne selektiv zu TBA umgesetzt wird.

Wird der Isobuten-haltige Stoffstrom mit Wasser in die Reaktivdestillationskolonne unterhalb der reaktiven Packung eingespeist, so steigen die leichtsiedenden C₄-Kohlenwasserstoffe dampfförmig in die Reaktionszone auf und aufgrund des Minimum-Azeotrop von Wasser und C₄-Kohlenwasserstoffen wird mit dem Edukt auch ein Teil des Wassers dampfförmig in die Reaktionszone transportiert. Das gegebenenfalls im Einsatzstoffgemisch enthaltene TBA und Teile des Wassers verbleiben im Sumpf und werden abgeführt.

Die Reaktivdestillationskolonne enthält in der Verstärkersäule den Katalysator, unter- und oberhalb der Katalysatorpackung können sich Trennböden und/oder Destillationspackungen befinden. Der Katalysator ist entweder in einer Packung integriert, beispielsweise KataMax^{®} (EP 0 428 265), KataPak^{®} (EP 0 396 650 oder DE 298 7 007.3), oder auf Formkörper aufpolymerisiert (US 5,244,929). Bevorzugt werden katalytische Packungen mit einem hohen Katalysatorgehalt eingesetzt, wie z. B. Katapak-SP 12 oder besonders bevorzugt Katapak-SP 11.

Unter dem Oberbegriff der Reaktivdestillation fallen alle verfahrenstechnischen Maßnahmen, bei denen Destillation und Reaktion stofflich und energetisch integriert durchgeführt werden. In den beschriebenen Katalysatorpackungen ist dies durch die Immobilisierung der Packungen in der Kolonne erreicht. Es ist im erfindungsgemäßen Verfahren auch möglich, diese Bereiche räumlich zu trennen, ohne auf die Vorteile einer Reaktivdestillation zu verzichten.

In einer Verfahrensvariante wird die Reaktivdestillationskolonne als Destillationskolonne mit einem oder mehreren außenliegenden Reaktoren, die den Katalysator enthalten und im Nebenstrom betrieben werden, ausgeführt.

Die Reaktivrektifikationskolonne, in der Isobuten umgesetzt und in der ein TBA-reicher Strom als Sumpfprodukt abgezogen wird, hat vorzugsweise eine Trennstufenzahl von 2 bis 60 insbesondere von 3 bis 50. Dabei entfallen vorzugsweise 5 bis 58 Trennstufen auf den Abtriebsteil, 2 bis 55 Trennstufen auf die Reaktionszone und 0 bis 20 Trennstufen auf den Verstärkerteil über der Reaktionszone. Zusätzliches Wasser kann an allen Stellen in die Kolonne eindosiert werden. Bevorzugt werden Wasser und vorreagiertes Gemisch an gleicher Position eingeleitet.

Der Betriebsdruck der Reaktivdestillationskolonne, gemessen am Kolonnenkopf, liegt zwischen 3 und 30 bara, insbesondere zwischen 4 und 12 bara. Das Rücklaufverhältnis liegt im Bereich von 0,5 bis 40, insbesondere im Bereich von 0,9 bis 20.

### Ausführungsform 2: Umsetzung von Isobuten mit Alkohol in Schritt a)

In einer bevorzugten Ausführungsform 2) des erfindungsgemäßen Verfahren wird in Schritt a) das Isobuten aus dem Isobuten-haltigen technischen Kohlenwasserstoffgemisch unter saurer Katalyse mit Alkohol zu Alkyl-tert.-butylether (ATBE, auch bezeichnet als tert.-Butyl-alkyl-ether) umgesetzt. Bei dieser Ausführungsform des erfindungsgemäßen Verfahren kann die Umsetzung von Isobuten mit Alkohol über 90 %, vorzugsweise über 95 % betragen. Als Alkohol können insbesondere Methanol oder Ethanol eingesetzt werden.

Die Veretherung des Isobutens wird als sauer katalysierte Reaktion durchgeführt. Als Alkohole können primäre, sekundäre, ein- oder mehrwertige Alkohole vorzugsweise mit 1 bis 5 C-Atomen, besonders bevorzugt Methanol oder Ethanol eingesetzt werden. Als Alkohol kann hochreiner Alkohol, reiner Alkohol oder Alkohol verwendet werden, der geringe Mengen an Verunreinigungen aufweist. Bevorzugt liegt die Reinheit des eingesetzten Alkohols, angegeben in Massen-% an Alkohol, über 90 %, besonders bevorzugt über 95 %, ganz besonders bevorzugt über 99 %. Der Gehalt an Wasser liegt bevorzugt unter 3 Massen-%, besonders bevorzugt unter 1 Massen-%, ganz besonders bevorzugt unter 0,3 Massen-%. Die Entwässerung des Ethanols kann auf herkömmliche Weise durch Azeotrop-Destillation oder durch Membranverfahren erfolgen.

Für die Umsetzung von Isobuten mit Alkoholen, insbesondere mit Methanol zu Methyl-tert.-butylether wurden diverse Verfahrensvarianten entwickelt (vergleich: Ullmann's Encyclopedia of Industrial Chemistry, Online Version, 2004, Wiley & Sons, Stichwort Methyl tert-Butyl Ether, und dort zitierte Literatur; Obenaus, Fritz; Droste, Wilhelm, Erdoel & Kohle, Erdgas, Petrochemie (1980), 33(6), 271-275; DE 2629769; DE 2853769). Prinzipiell sind alle Verfahren zur Umsetzung des Isobutens mit Alkoholen im Rahmen dieser Erfindung als Verfahrenschritt a) geeignet. Bevorzugt werden Verfahren, bei denen die Umsetzung in flüssiger Phase an einem sauren Ionenaustauscherharz erfolgt. Die Umsetzung von Isobuten mit Alkohol kann wie in Verfahrensschritt d) beschrieben erfolgen. Als Reaktoren, in denen der Alkohol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren etc.) eingesetzt werden. Sie können mit oder ohne partieller Rückführung, wobei gegebenenfalls der Rückführungsstrom gekühlt werden kann, betrieben werden.

In einer bevorzugten Ausführungsform wird die Umsetzung des Isobutens mindestens zweistufig durchgeführt, wobei die erste Stufe als adiabatischer Festbettreaktor mit Rückführung und die folgenden Stufen als Festbettstufen ohne Rückführung und/oder als Reaktivdestillation betrieben werden. Das Verhältnis von rückgeführter Menge zu Frischzulauf (C₄-Kohlenwasserstoffe und Alkohol) liegt bevorzugt bei 0,5 bis 20 t/t, besonders bevorzugt bei 1 bis 5 t/t. Die Reaktoren können bei Temperaturen von vorzugsweise 10 bis 160 °C, bevorzugt von 30 bis 110 °C, betrieben werden. Der Druck in den Festbettstufen beträgt vorzugsweise 5 bis 50 bara, bevorzugt 10 bis 20 bara. Da das thermodynamische Gleichgewicht zwischen Alkohol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben.

Das molare Verhältnis von Alkohol zu Isobuten beträgt bei dieser Ausführungsform des erfindungsgemäßen Verfahrensschrittes a) vorzugsweise von 5 zu 1 bis 0,9 zu 1, bevorzugt von 2 zu 1 bis 1 zu 1 und besonders bevorzugt von 1,5 zu 1 bis 1 zu 1. Da im Verfahrensschritt a) ein geringerer Umsatz an Isobuten akzeptiert werden kann, kann im Vergleich zu Verfahrensschritt d) ein geringerer Alkoholüberschuss vorteilhaft sein.

In einer bevorzugten Ausführungsform wird die Addition des Alkohols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt a) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus dem Isobuten-haltigen technischen Kohlenwasserstoffgemisch I und Alkohol ein Reaktionsgemisch hergestellt, das hinsichtlich seiner Isobuten-, Alkohol- und tert.-Butylether-Konzentration in der Nähe des thermodynamischen Gleichgewichts liegt. Der Umsatz des Isobutens beträgt dabei bevorzugt größer 90 %. Dieses Gemisch wird in der nächsten/letzten Reaktionsstufe in die Reaktivdestillationskolonne eingespeist, wo ein weiterer Teil des Isobutens zum Ether umgesetzt wird.

Als Katalysator wird sowohl in den Festbettstufen als auch in der Reaktivdestillationskolonne ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen keine sauren Stoffe an das Produktgemisch abgeben, weil dies zu Ausbeuteverlusten führen kann. Als Katalysatoren können bevorzugt solche eingesetzt werden, wie sie im Verfahrensschritt d) beschrieben werden, wobei das erfindungsgemäße Verfahren so durchgeführt werden kann, dass in Schritt a) und d) jeweils der gleiche Katalysator oder unterschiedliche Katalysatoren eingesetzt werden können. Vorzugsweise werden in Schritt a) und d) die gleichen Katalysatoren eingesetzt.

In der Reaktivdestillationskolonne kann der Katalysator entweder in der Packung integriert sein, beispielsweise KataMax^{®} (wie in EP 0 428 265 beschrieben), KataPak^{®} (wie in EP 0 396 650 oder DE 298 07 007.3 beschrieben) oder auf Formkörpern aufpolymerisiert (wie in US 5,244,929 beschrieben) sein.

Die Umsetzung des Isobutens mit Alkohol zum entsprechenden tertiären Butylether erfolgt in der Reaktivdestillation vorzugsweise im Temperaturbereich von 10 bis 140 °C, bevorzugt von 40 bis 90 °C, besonders bevorzugt von 60 bis 80 °C (Bereich der Kolonne, in der sich der Katalysator befindet. Die Sumpftemperatur der Kolonne kann deutlich höher liegen).

Insbesondere wird das Isobuten durch Umsetzung mit Alkohol (Methanol, Ethanol) zu ATBE (MTBE, ETBE) entfernt. Dabei wird insbesondere derart verfahren, wie in DE 101 02 082 für MTBE beschrieben. Das Isobuten aufweisende C₄-Kohlenwasserstoffgemisch wird zusammen mit Alkohol in den Vorreaktor eingespeist. Dabei wird der Alkohol bevorzugt im Überschuss eingesetzt. In den Vorreaktoren entsteht ein Gemisch, in dem Isobuten, Alkohol und ATBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Alkohol enthalten sein, als für die vollständige Umsetzung des noch vorhandenen Isobutens gebraucht wird. Der Alkoholüberschuss sollte jedoch derart bemessen werden, dass eine ausreichende Alkoholmenge für das sich bildende Azeotrop aus Alkohol und C₄-Kohlenwasserstoffen vorhanden ist.

Optional kann, beispielsweise wenn der Alkoholgehalt im Kolonnenzulauf zur Reaktivdestillationskolonne unter dem maximal zulässigen Wert liegt, zusätzlicher Alkohol zugemischt werden. Darüber hinaus kann am Kopf der Reaktivdestillationskolonne unterhalb oder in einem Flüssigkeitsverteiler über eine gesonderte Einrichtung eine Alkohol-Einspeisung erfolgen.

Bevorzugt weist die Reaktivdestillationskolonne oberhalb der Katalysatorpackung einen Bereich rein destillativer Trennung auf, besonders bevorzugt mit 5 bis 20, insbesondere mit 10 bis 15 Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators umfasst 12 bis 36, insbesondere 20 bis 30 Trennstufen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 75 bis 99 %, vorzugsweise von 85 bis 98 % und besonders bevorzugt von 95 bis 97 % bezogen auf den Isobutengehalt im Zulauf zur Reaktivdestillation erreicht wird.

Die mittlere Temperatur in der Katalysatorzone beträgt abhängig vom Druck in der Kolonne vorzugsweise 55 °C bis 70 °C, besonders bevorzugt 58 °C bis 67 °C. Die Reaktivdestillationskolonne wird bei Drücken, gemessen am Kolonnenkopf, von 3 bara bis 15 bara, bevorzugt 5 bara bis 11 bara betrieben, insbesondere von 7 bara bis 10 bara.

Die hydraulische Belastung in der katalytischen Packung der Kolonne beträgt bevorzugt 10 % bis 110 %, vorzugsweise 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden (Vauck/Müller, ,,Grundoperationen chemischer Verfahrenstechnik", S. 626, VEB Deutscher Verlag für Grundstoffindustrie.). Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen kleiner als 1,5 betrieben, insbesondere mit solchen, die größer 0,4 und kleiner 1, bevorzugt größer 0,5 und kleiner 0,9 betragen.

Das Sumpfprodukt der Reaktivdestillationskolonne besteht vorzugsweise hauptsächlich aus ATBE. Bevorzugt enthält es weniger als 2500 wppm Alkyl-sec.-butylether und weniger als 2500 wppm C₈-Kohlenwasserstoffe.

Das Kopfprodukt der Reaktivdestillation kann wiederum in ein C₄-Kohlenwasserstoffgemisch und Alkohol getrennt werden, wobei das C₄-Kohlenwasserstoffgemisch bevorzugt weniger als 0,5 wppm ATBE und/oder TBA enthält.

Der Alkohol kann z. B. durch Extraktion mit Wasser abgetrennt werden. Falls Spuren von Butadien nicht schon vor dem Verfahrensschritt a) entfernt wurden, können sie aus dem so erhaltenen Raffinat II durch Selektivhydrierung (SHP) entfernt werden (vgl. Erdoel & Kohle, Erdgas, Petrochemie (1986), 39(2), 73-8). Das aus der Reaktivdestillation erhaltene und gegebenenfalls von Alkohol befreite C₄-Kohlenwasserstoffgemisch kann dem Verfahrenschritt c) zugeführt werden.

Werden für die Veretherung andere Alkohole als Methanol oder Ethanol eingesetzt, so ändern sich die Parameter der Reaktivdestillation entsprechend.

Der bei dieser Ausführungsart des erfindungsgemäßen Verfahrens anfallende ATBE kann ebenso den unter Verfahrenschritt d) beschriebenen Verwendungszwecken zugeführt werden.

Wird in Schritt a) Isobuten mit Alkohol umgesetzt ohne dass eine Reaktivdestillation eingesetzt wurde, werden in Stufe b) in einem ersten Schritt C₄-Kohlenwasserstoffe gegebenenfalls zusammen mit Restmengen Alkohol vom Produkt II, vorzugsweise destillativ, abgetrennt und, falls erforderlich, in einem zweiten Schritt der Alkohol extraktiv von den C₄-Kohlenwasserstoffen III abgetrennt. Wird in Schritt a) eine Reaktivdestillationskolonne als letzte Verfahrensstufe durchgeführt, so kann auf die destillative Trennung in Schritt b) verzichtet werden und das bei der Reaktivdestillation erhaltene Kopfprodukt aus gegebenenfalls vorhandenem nicht umgesetzten Alkohol und nicht umgesetztem C₄-Kohlenwasserstoffen kann entweder direkt einer Extraktion zur Entfernung des Alkohols oder Schritt c) zugeführt werden. Die Extraktion kann so ausgeführt sein, wie in Verfahrensschritt e) beschrieben.

### Ausführungsform 3: Oligomerisierung von Isobuten als Umsetzung in Schritt a)

In einer weiteren bevorzugten Ausführungsform des erfindungsgemäßen Verfahrens wird in Schritt a) das Isobuten aus dem Isobuten-haltigen technischen Kohlenwasserstoffgemisch zu Isobutenoligomeren, insbesondere zu Diisobuten umgesetzt. Isobutenoligomere im Sinne der vorliegenden Erfindung sind insbesondere Isobutenoligomere wie Di-, Tri- oder Tetramere des Isobutens. Zudem können diese auch Cooligomere mit oder von 1- oder 2-Butenen enthalten.

Die partielle Oligomerisierung in Schritt a) des Isobutens kann prinzipiell homogen, d. h. unter Verwendung von im Reaktionsgemisch löslichen Katalysatoren, oder heterogen, d. h. unter Verwendung von im Reaktionsgemisch unlöslichen Katalysatoren durchgeführt werden. Der Nachteil der homogenen Verfahren besteht darin, dass der Katalysator den Reaktor mit den Reaktionsprodukten und nicht umgesetzten Edukten verlässt, von denen er abgetrennt, aufgearbeitet und entsorgt oder zurückgeführt werden muss.

Wegen dieses hohen Trennaufwands wird die partielle Oligomerisierung des Isobutens in Schritt a) bevorzugt an festen heterogenen Katalysatoren, die zudem oft im Festbett angeordnet sind, durchgeführt, so dass eine aufwändige Katalysatorabtrennung entfällt.

Als Festkatalysatoren können saure Stoffe eingesetzt werden, die im Edukt/Produktgemisch unlöslich sind. Die meisten dieser Katalysatoren gehören einer der folgenden Gruppen an:
a) Mineralsäuren (z. B. Schwefelsäure oder Phosphorsäure) auf einem Trägermaterial (z. B. Aluminiumoxid oder Siliciumdioxid),
b) Zeolithe oder andere Alumosilikate mit oder ohne Dotierung weiterer Metalle, insbesondere mit Übergangsmetallen oder
c) Saure Ionenaustauscherharze, insbesondere saure Kationenaustauscher.

Wegen der höheren Selektivität für die Bildung von Isobutenoligomeren und wegen der geringeren Bildung von Nebenprodukten werden bevorzugt saure Ionenaustauscherharze als Katalysator verwendet. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomeren sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Eigenschaften dieser Harze, insbesondere spezifische Oberfläche, Porosität, Stabilität, Quellung bzw. Schrumpfung und Austauschkapazität, können durch den Herstellprozess variiert werden. Die Harze können gelförmig, makroporös oder schwammförmig hergestellt werden. Stark saure Harze des Styrol-Divinylbenzol-Typs werden u. a. unter folgenden Handelsnamen verkauft: CT 151 der Firma Purolite, Amberlyst 15, Amberlyst 35, Amberlite IR-120, Amberlite 200 der Firma Rohm&Haas, Dowex M-31 der Firma DOW, K 2611, K 2431 der Firma Bayer.

Die Ionenaustauscherkapazität der vollständig in der H⁺-Form vorliegenden Harze beträgt vorzugsweise von 1 bis 2, insbesondere von 1,5 bis 1,9 Mol H⁺ pro Liter feuchtes Harz (handelsüblich). Im Verfahren der Erfindung werden vorzugsweise makroporöse Harze eingesetzt, wie beispielsweise K 2431 der Firma Bayer, Amberlyst 15 oder Amberlyst 35 der Firma Rohm & Haas. Das Porenvolumen beträgt bevorzugt 0,3 bis 0,6 ml/g, insbesondere 0,4 bis 0,5 ml/g (bezogen auf handelsübliches wasserfeuchtes Harz). Die Korngröße von im erfindungsgemäßen Verfahren in Schritt a) vorzugsweise einzusetzendem Harz liegt im Bereich von 500 µm bis 1500 µm, insbesondere von 600 µm bis 1000 µm. Die Korngrößenverteilung kann enger oder weiter gewählt werden. So können beispielsweise Ionenaustauscherharze mit sehr einheitlicher Korngröße (monodisperse Harze) eingesetzt werden.

Es kann vorteilhaft sein, in Reaktoren, die mit hohen Lineargeschwindigkeiten durchströmt werden, zur Verringerung des Differenzdruckes ein monodisperses Korn, und in Reaktoren, die mit einer geringen Lineargeschwindigkeit durchströmt werden, zur Erzielung des optimalen Umsatzes ein Korn mit breiter Korngrößenverteilung einzusetzen. Optional können die Ionenaustauscherharze als Formkörper, wie beispielsweise Zylinder, Ringe oder Kugeln, eingesetzt werden.

Das saure Ionenaustauscherharz wird zweckmäßig auf eine Aktivität eingestellt, die zwar die Oligomerisierung des Isobutens ermöglicht, jedoch die Cooligomerisierung von Isobuten mit linearen Butenen, die Oligomerisierung der linearen Butene sowie die Isomerisierung der linearen Butene kaum katalysiert. Weiterhin wird die Wärmeentwicklung damit im Reaktor auf einen technisch gut beherrschbaren Wert eingestellt.

Die Einstellung der gewünschten Katalysatoraktivität kann mit Hilfe von Moderatoren geschehen. Diese Stoffe werden zusammen mit dem Edukt über den Katalysator geleitet. Als Moderator können beispielsweise Wasser, Alkohole wie tert.-Butylalkohol (TBA), Methanol, Isononanol oder Ethanol oder Ether wie MTBE oder ETBE jeweils als Reinstoff oder Gemische eingesetzt werden. Die Oligomerisierung in Stufe a) wird daher bevorzugt in Gegenwart dieser Moderatoren durchgeführt. Bei der Verwendung von Moderatoren werden vorzugsweise Molverhältnisse von 0,01 bis 5, bevorzugt 0,01 bis 1, insbesondere 0,01 bis 0,7 Mol Moderator pro Mol Isobuten eingestellt.

Im erfindungsgemäßen Verfahren können in Schritt a) zur Oligomerisation weiterhin feste sulfonierte Kationenaustauscher(-harze) eingesetzt werden, die ohne Zusatz von Moderatoren die gewünschte Aktivität aufweisen. Dies sind insbesondere teilneutralisierte Kationenaustauscher, bei denen ein Teil der aciden Wasserstoffatome der Sulfonsäuregruppen gegen Metallionen, insbesondere Metallionen der Elemente der 1. bis 12. Gruppe des Periodensystems, ausgetauscht wurden. Vorzugsweise werden Kationenaustauscher eingesetzt, bei denen 1 bis 70 %, bevorzugt 5 bis 50 %, ganz besonders bevorzugt 10 bis 40 % der aciden Wasserstoffatome der Sulfonsäuregruppen gegen Metallionen ausgetauscht worden sind. Als Metallionen, die die Wasserstoffatome ersetzen, können insbesondere Alkalimetall-, Erdalkalimetall-, Übergangsmetallionen, wie z. B. Chrom-, Mangan-, Eisen-, Kobalt-, Nickel-, Zinkionen und Aluminiumionen sowie Ionen der Lanthanidgruppe (Seltene Erden) verwendet werden. Bevorzugt werden die aciden Wasserstoffatome durch Alkalimetallionen, insbesondere Natriumionen ersetzt.

Es ist auch möglich, dass das Ionenaustauscherharz mit zwei oder mehreren unterschiedlichen Metallionen beladen ist.

Für die Herstellung der teilneutralisierten Ionenaustauscherharze können verschiedene Verfahren, die alle in der Fachliteratur beschrieben werden, angewendet werden. Solche Verfahren werden z. B. in EP 1 388 528 beschrieben.

Ein Reaktor in dem erfindungsgemäßen Verfahren kann ein Gemisch von Ionenaustauscherharzen unterschiedlicher Reaktivität enthalten. Ebenso ist es möglich, dass ein Reaktor Katalysatoren mit unterschiedlicher Aktivität, z. B. in Schichten angeordnet, enthält. Wird mehr als ein Reaktor verwendet, können die einzelnen Reaktoren mit Katalysatoren gleicher oder unterschiedlicher Aktivität gefüllt sein.

Für die technische Ausführung der Umsetzung der Isobuten-haltigen Kohlenwasserstoffmischungen sind diverse Varianten möglich. Die Umsetzung kann chargenweise oder bevorzugt in kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Ein anderes Konzept als Festbettreaktoren sind beispielsweise Reaktoren, in denen der Ionentauscher suspendiert in einer flüssigen Phase vorliegt (vergleiche "Bayer Verfahren", Erdöl und Kohle, Erdgas, Petrochemie, 1974, 27, Heft 5, Seite 240).

Wenn ein Festbett-Strömungsreaktor verwendet wird, kann die Flüssigkeit aufwärts oder abwärts strömen. Meistens wird ein Abwärtsströmen der Flüssigkeit bevorzugt. Eine den Reaktor umströmende Kühlflüssigkeit kann gegebenenfalls gleich oder entgegengesetzte Strömungsrichtung aufweisen. Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden.

Die im technischen Prozess eingesetzten Reaktoren können adiabatisch, polytrop oder praktisch isotherm betrieben werden. Praktisch isotherm bedeutet, dass die Temperatur an einer beliebigen Stelle im Reaktor maximal um 10 °C höher ist als die Temperatur am Reaktoreingang. Bei adiabatischem Betrieb der Reaktoren ist es in der Regel sinnvoll, mehrere Reaktoren in Reihe zu schalten und vorzugsweise zwischen den Reaktoren zu kühlen. Reaktoren, die für einen polytropen oder praktisch isothermen Betrieb geeignet sind, sind beispielsweise Rohrbündelreaktoren, Rührkessel und Schlaufenreaktoren. Es ist möglich, mehrere Reaktoren, auch verschiedene Bauarten, zu kombinieren. Es ist zudem möglich, Reaktoren unter Rückführung von Produkt zu betreiben.

Die Temperaturen, bei denen die Oligomerisation betrieben wird, können von 15 bis 160 °C, vorzugsweise von 40 bis 110 °C betragen.

Die Umsetzung kann mit und ohne Zugabe eines zusätzlichen Lösungsmittels erfolgen. Als Lösungsmittel werden bevorzugt gesättigte Kohlenwasserstoffe eingesetzt, insbesondere C₄-, C₈-oder C₁₂ Kohlenwasserstoffe. Ganz besonders bevorzugt ist der Einsatz von Isooktan. Bei Zugabe von Lösungsmitteln beträgt ihr Anteil 0 bis 60 Massen-%, bevorzugt 0 bis 30 Massen-%.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar, d. h. die Isobuten-haltigen Kohlenwasserstoffmischungen liegen während der Oligomerisation ganz oder teilweise in flüssiger Phase vor. Wenn die Reaktion vollständig in der Flüssigphase durchgeführt werden soll, sollte der Druck 2 bis 4 bar vorzugsweise höher als der Dampfdruck des Reaktionsgemisches sein, um in den Reaktoren Verdampfungsprobleme zu vermeiden.

Auch wenn die Reaktion bei einem Druck betrieben wird, bei dem die Reaktionsmischung nicht vollständig flüssig vorliegt (beispielsweise in einer Reaktivdestillation oder bei Verfahrensvarianten analog US 5,003,124), findet die Oligomerisierung nach dem erfindungsgemäßen Verfahren trotzdem in der Flüssigphase, also an "feuchtem" d. h. mit Flüssigkeit benetztem Katalysator statt.

Der Gesamtumsatz an Isobuten zu Oligomeren kann über die Art und Menge des verwendeten Katalysators, die eingestellten Reaktionsbedingungen und Anzahl der Reaktoren eingestellt werden. Im erfindungsgemäßen Verfahren werden vorzugsweise 30 bis 95 %, bevorzugt 50 bis 80 %, besonders bevorzugt 55 bis 70 %, des im Edukt enthaltenden Isobutens oligomerisiert.

Das Reaktionsgemisch der partiellen Isobuten-Oligomerisierung kann unterschiedlich aufgearbeitet werden. Die Abtrennung der Produkte II (Oligomere) und gegebenenfalls von Kohlenwasserstoffen mit 5 bis 7 Kohlenstoffatomen von nicht umgesetzten C₄-Kohlenwasserstoffen III gemäß Schritt b) erfolgt zweckmäßig durch Destillation. Bevorzugt wird die Destillation bei einem Druck von 1 bis 10 bara, besonders bevorzugt bei einem Druck von 4 bis 7 bara betrieben. Die Temperaturen im Sumpf betragen vorzugsweise von 120 bis 220 °C, besonders bevorzugt von 170 bis 200 °C. Das Rücklaufverhältnis wird vorzugsweise auf Werte von 0,1 bis 1,5, vorzugsweise von 0,3 bis 1,0 eingestellt. Die Destillation wird vorzugsweise in einer Kolonne mit einer Anzahl an Böden von 20 bis 40, bevorzugt 25 bis 35 durchgeführt. Die so abgetrennten nicht umgesetzten C₄-Kohlenwasserstoffe werden im erfindungsgemäßen Schritt c) weiterbehandelt.

Die abgetrennte Oligomerenfraktion enthält hauptsächlich C₈-Kohlenwasserstoffe und kann gegebenenfalls die eingesetzten Moderatoren aufweisen. Sie kann neben dem Diisobuten auch Codimere und höhere Oligomere (C₁₂, C₁₆₊,) enthalten. Der Anteil an Cooligomeren liegt bevorzugt unter 50 Massen-%, besonders bevorzugt unter 25 Massen-%. Diese Fraktion kann in weiteren Destillationsschritten aufgetrennt werden. So ist es beispielsweise möglich, eine Fraktion mit hochreinem Diisobuten abzutrennen, um diese separat, beispielsweise für chemische Synthesen, einzusetzen. Für den Einsatz als Kraftstofftkomponente für Ottomotoren kann es notwendig sein, hochsiedende Komponenten (vorzugsweise Siedepunkt > 220 °C) abzutrennen.

Es ist auch möglich, die Butenoligomeren, insbesondere die C₈-Olefine, ganz oder teilweise zu hydrieren. Methoden zur Hydrierung der Produkte der Oligomerisierung zu den entsprechenden Paraffinen sind dem Fachmann hinlänglich bekannt. Gängige Methoden zur Hydrierung von Olefinen sind beispielsweise beschrieben in F. Asinger, ,,Chemie und Technologie der Monoolefine", Akademie Verlag, Berlin, 1957, Seite 626 - 628 oder DE 197 19 833.

In einer bevorzugten Ausführungsform wird die Hydrierung in flüssiger Phase an einem festen, im Hydriergut nicht löslichen Katalysator durchgeführt. Als Hydrierkatalysatoren werden bevorzugt Trägerkatalysatoren, die aus einem anorganischen Träger bestehen und als Aktivmetall Platin und/oder Palladium und/oder Nickel enthalten, verwendet. Die Temperatur, bei der die Hydrierung durchgeführt wird, liegt bevorzugt im Bereich von 10 bis 250 °C und der Druck zwischen 1 und 100 bar.

Nach der Hydrierung können durch destillative Trennung weitere Fraktionen gewonnen werden. Aus diesen und aus den unhydrierten Fraktionen sind durch Abmischung Kraftstoffadditive bestimmter Eigenschaften erhältlich. Weiterhin können einige Fraktionen als Lösemittel verwendet werden.

### Ausführungsform 4: Umsetzung von Isobuten in Schritt a) mit Formaldehyd

In einer weiteren bevorzugten Ausführungsform des erfindungsgemäßen Verfahren wird im Schritt a) das Isobuten mit Formaldehyd oder einem Formaldehyd Derivat umgesetzt. Dabei werden je nach Reaktionsbedingungen 4,4-Dimethyl-1,3-Dioxan oder 3-Methyl-3-buten-1-ol als Hauptprodukt erhalten. Das Verfahren ist seit langem in der Literatur bekannt. Informationen zu diesem Verfahren sind beispielsweise in der aktuellen Ausgabe von Ullmann's Encyclopedia of Industrial Chemistry, Wiley-VCH-Verlag unter dem Stichwort "Isopren" sowie den dort genannten Literaturstellen, in Weissermel, Arpe, Industrielle Organische Chemie, VCH, 4. Auflage 1994, Seiten 127 bis 131 und in W. Swodenk, W. Schwerdtel, P. Losacker, Erdöl und Kohle - Erdgas - Petrochemie, 1970, 23, 641 bis 644 zu finden. Die beiden Produkte 3-Methyl-3-buten-1-ol oder 4,4-Dimethyl-1,3-Dioxan (1,3-Dioxan) werden hauptsächlich für die Herstellung von Isopren eingesetzt. Das durch Umsetzung von Isobuten mit Formaldehyd erhältliche 1,3-Dioxan kann beispielsweise durch Spaltung in der Gasphase bei 200 bis 300 °C über einem sauren Katalysator, z. B. Phosphorsäure auf einem Träger, zu Isopren umgesetzt werden, wobei die Hälfte des zur Herstellung des 1,3-Dioxans benötigte Menge an Formaldehyd wieder zurückgewonnen werden kann (vgl. DE 196 31 005).

Das 3-Methyl-3-buten-1-ol, welches auch aus dem 1,3-Dioxan durch Abspaltung von Formaldehyd erhalten werden kann, kann beispielsweise auch zur Darstellung von 3-Methylbutan-1-ol eingesetzt werden, aus dem durch Wasserabspaltung wiederum 3-Methyl-1-buten erhalten werden kann.

Die Umsetzung des Isobutens kann entweder direkt mit dem Formaldehyd, gegebenenfalls als Lösung von Formaldehyd in Wasser oder einem anderen Lösungsmittel, oder mit einem geeigneten Formaldehyd-Derivat erfolgen. Geeignete Formaldehyd-Derivate sind beispielsweise Methylal oder Dioxolan. Die Umsetzung kann mit oder ohne Einsatz von Katalysatoren erfolgen und ist sowohl in der Flüssigphase sowie in der Gasphase möglich (vergleiche DE 15 93 851; DE 17 68 057; DE 12 75 049; US 2,308,192; FR 155 6915; Studies in Surface Science and Catalysis, Vol. 125, 199, 507 bis 514). Bevorzugt wird die Umsetzung in der Flüssigphase unter Einsatz von Katalysatoren durchgeführt. Als Katalysatoren werden bevorzugt Metallkatalysatoren, Brönstedt- und Lewissäuren eingesetzt. Besonders bevorzugt eingesetzt werden Übergangsmetallkatalysatoren und saure Kationenaustauscherharze, wie sie auch in der Ausführungsform 1 des Verfahrensschritts a) eingesetzt werden.

Der bei der Umsetzung mit Formaldehyd erzielte Isobutenumsatz liegt bevorzugt bei 30 bis 90 %, besonders bevorzugt bei 40 bis 70 %.

Das Reaktionsgemisch der Reaktion gemäß der Ausführungsform 4 des Schrittes a) wird je nach Art der gewählten Umsetzung unterschiedlich aufgearbeitet. Liegen nach der Reaktion mehrere Phasen vor, beispielsweise eine wässrige Phase und eine organische, die hauptsächlich die nicht umgesetzten C₄-Kohlenwasserstoffe und die Hauptprodukte der Umsetzung enthält, wird zuerst eine Phasentrennung durchgeführt. Zur Entfernung von Restmengen an Formaldehyd kann anschließend eine Wasserwäsche erfolgen. Die Abtrennung der Produkte II (Allylalkohole, 1,3 Dioxane oder 1,3-Diole) und gegebenenfalls von Kohlenwasserstoffen mit 5 bis 7 Kohlenstoffatomen von nicht umgesetzten C₄-Kohlenwasserstoffen III gemäß Schritt b) erfolgt anschließend zweckmäßig durch Destillation. Dabei werden zumindest in einer Destillationskolonne die nicht umgesetzten C₄-Kohlenwasserstoffe als Kopfprodukt erhalten. Diese Destillation erfolgt vorzugsweise bei einem Druck von 1 bis 11 bara, besonders bevorzugt bei 3 bis 8 bara und ganz besonders bevorzugt 4 bis 7 bara. Die Temperatur im Kopf der Kolonne beträgt vorzugsweise von 40 bis 60 °C, bevorzugt von 45 bis 50 °C. Bei der Destillation wird bevorzugt ein Rücklaufverhältnis von 0,5 bis 2, vorzugsweise von ca. 1 eingestellt. Fällt am Kopf der Kolonne neben den nicht umgesetzten C₄-Kohlenwasserstoffe eine zweite, wässrige Phase an, wird diese bevorzugt in einem Kopfdekanter abgetrennt. Die Destillation wird vorzugsweise in einer Kolonne durchgeführt, die mindestens eine Anzahl von 20 theoretischen Böden, vorzugsweise von 25 bis 50 und besonders bevorzugt von 30 bis 40 theoretischen Böden aufweist. Die so abgetrennten nicht umgesetzten Kohlenwasserstoffe III werden im erfindungsgemäßen Schritt c) weiterbehandelt.

Die bei der Destillation anfallenden Fraktionen, die 3-Methyl-3-buten-1-ol und/oder 4,4-Dimethyl-1,3-Dioxan enthalten können, können direkt oder nach weiterer Reinigung, z. B. einer weiteren Destillation oder Extraktion, wie oben beschrieben weiter zu Isopren umgesetzt werden.

### Einsatzstoffe

In dem erfindungsgemäßen Verfahren können alle üblicherweise zur Verfügung stehenden technischen C₄-Kohlenwasserstoffgemische eingesetzt werden. Geeignete Isobuten-haltige C₄-Ströme sind beispielsweise Leichtbenzinfraktionen aus Raffinerien, C₄-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben. (K. Weissermel, H. J. Arpe, Industrielle Organische Chemie, Wiley-VCH, 5. Auflage, 1998, Seiten 23 bis 24; 65 bis 99; 122 bis 124).

Bevorzugt eingesetzt werden C₄-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, oder C₄-Fraktionen aus Katcrackern. Die als Nebenprodukt anfallenden C₄-Schnitte enthalten je nach Crack-Verfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der C₄-Fraktion liegen bei C₄-Fraktionen aus Steam-Crackern bei 18 bis 35 Massen-%, bei Fluid-Katcrackem (FCC) bei 10 bis 20 Massen-%.

Für das erfindungsgemäße Verfahren ist es vorteilhaft, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seiten 119 bis 121).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 0 523 482. Auch durch selektive Umsetzungen des 1,3-Butadiens, zum Beispiel Dimerisierung zum Cyclooctadien, Trimerisierung zum Cyclododecadien, Polymerisations- oder Telomerisationsreaktionen, kann das 1,3-Butadien zumindest teilweise entfernt werden. Wurde ein Crack-C₄-Schnitt als Rohstoff eingesetzt, bleibt in allen Fällen ein Kohlenwasserstoffgemisch (z. B. Raffinat I oder hydriertes Crack-C₄ (HCC₄)) zurück, das hauptsächlich die gesättigten Kohlenwasserstoffe, n-Butan und Isobutan und die Olefine Isobuten, 1-Buten und 2-Butene enthält.

Bevorzugt werden in dem erfindungsgemäßen Verfahren in einer zusätzlichen Reinigungsstufe, die einer oder mehreren der Verfahrensschritte a), b), c), d), e) oder f) vorgeschaltet wird, in den C₄-Kohlenwasserstoffströmen enthaltene mehrfach ungesättigten Kohlenwasserstoffe katalytisch selektiv hydriert. Besonders bevorzugt ist zumindest vor dem Verfahrensschritt a) und/oder c) eine solche Reinigungsstufe vorgesehen, insbesondere wenn nicht ausgeschlossen werden kann, dass die eingesetzten technischen C₄-Kohlenwasserstoffströme mehrfach ungesättigte Kohlenwasserstoffe aufweisen.

Bei den mehrfach ungesättigten Kohlenwasserstoffen handelt es sich hauptsächlich um 1,3-Butadien; 1,2-Butadien, Butenin und 1-Butin sind, wenn überhaupt, in deutlich geringerer Menge enthalten. Die Hydrierung kann in einem ein- oder mehrstufigen Hydrierprozess in der Flüssigphase an einem Palladiumkontakt erfolgen. Zu Absenkung des Gehalts an 1,3-Butadien unter vorzugsweise 1000 wppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0,05 bis 100 Gew.-ppm (wppm) zugesetzt wird. Der Gehalt an mehrfach ungesättigten Kohlenwasserstoffen sollte im Zulauf zu dieser Stufe unter 1 %, bevorzugt unter 0,5 %, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986, 39, 73).

Sind in den Isobuten-haltigen C₄-Strömen Mengen größer 1 % an mehrfach ungesättigten Kohlenwasserstoffe wie 1,3-Butadien enthalten, werden sie vorzugsweise in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-C₄ aus einem Steam-Cracker mit einem Gehalt an 1,3-Butadien von typischerweise 38 bis 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer Selektivhydrierung (SHP) erfolgen kann.

Die in dem erfindungsgemäßen Verfahren eingesetzten Kohlenwasserstoffmischungen mit Isobuten und linearen Butenen weisen bevorzugt die folgenden Zusammensetzungen auf, wobei je nach Gehalt an ungesättigten Kohlenwasserstoffen eine Hydrierung oder Selektivhydrierung vor einem der Schritte a) bis d), vorzugsweise vor Schritt c) durchgeführt wird.

**Tabelle 1: Typische Zusammensetzungen von technischen Kohlenwasserstoffgemischen, die im erfindungsgemäßen Verfahren eingesetzt werden können.**

| | Steamcracker | | Steamcracker | | Katcracker | |
|---|---|---|---|---|---|---|
| Komponente | HCC₄ | HCC₄ / SHP | Raff. I | Raff. I / SHP | CC₄ | CC₄ / SHP |
| Isobutan [Massen-%] | 1 - 4.5 | 1 - 4.5 | 1.5 - 8 | 1.5 - 8 | 37 | 37 |
| n-Butan [Massen-%] | 5-8 | 5-8 | 6-15 | 6-15 | 13 | 13 |
| trans-Buten [Massen-%] | 18 - 21 | 18 - 21 | 7 - 10 | 7 - 10 | 12 | 12 |
| 1-Buten [Massen-%] | 35 - 45 | 35 - 45 | 15 - 35 | 15 - 35 | 12 | 12 |
| Isobuten [Massen-%] | 22 - 28 | 22 - 28 | 33 - 50 | 33 - 50 | 15 | 15 |
| cis-Buten [Massen-%] | 5 - 9 | 5 - 9 | 4-8 | 4-8 | 11 | 11 |
| 1,3-Butadien [wppm] | 500 - 8000 | 0-50 | 50 - 8000 | 0 - 50 | < 10000 | 0 - 50 |

### Erläuterung

- HCC₄: typisch für eine C₄ Mischung, die aus dem Crack-C₄ eines Steamcrackers (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einer SHP weiter reduziert wurden.
- Raff. I (Raffinat I): typisch für eine C₄ Mischung, die aus dem Crack-C₄ eines Steamcrackers (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einer SHP weiter reduziert wurden.
- CC₄: typische Zusammensetzung eines Crack-C₄, das aus einem Katcracker erhalten wird.
CC₄ / SHP: Zusammensetzung CC₄, bei dem Reste an 1,3-Butadien in einer SHP weiter reduziert wurden.

Das Raffinat I bzw. HCC₄ ist, neben anderen, ein bevorzugt eingesetztes Isobuten-haltiges Kohlenwasserstoffgemisch im Rahmen dieser Erfindung. Da Anlagen zur Aufarbeitung von C₄-Kohlenwasserstoffen in der Regel als Strang (Verbund mehrerer Anlagen) aufgebaut sind, ist es jedoch möglich, dass das Raffinat I bzw. HCC₄ vor dem Eintritt in das erfindungsgemäße Verfahren eine oder mehrere andere Prozessstufen durchläuft. Diese Prozessstufen können beispielsweise auch Verfahren bzw. Verfahrensschritte sein, wie sie in den Ausführungsformen zum Verfahrensschritt a) beschrieben worden sind. In dem erfindungsgemäßen Verfahren einsetzbare C₄-Kohlenwasserstoffgemische können auch solche sein, wie sie aus Verfahren gemäß den Ausführungsformen der Verfahrensschritt a) und anschließender Trennung gemäß Verfahrensschritt b) erhalten werden. Insbesondere können auch solche Gemische eingesetzt werden, wie sie bei der Herstellung von TBA aus Isobuten nach Abtrennung des TBA erhalten werden. Auf diese Weise kann jeweils ein individuell angepasstes Gesamtkonzept zur Aufarbeitung mit dem entsprechenden Produktportfolio realisiert werden.

Typische Verfahren, die den erfindungsgemäßen Verfahren vorgelagert sein können, sind Wasserwäschen, Reinigungsverfahren an Adsorptionsmitteln, Trocknungsverfahren und Destillationen.

### Wasserwäsche

Durch eine Wasserwäsche können hydrophile Komponenten aus dem einzusetzenden technischen Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestilltion stammen können). Auch Sauerstoffverbindungen (z. B. Aceton aus einer FCC-Einheit) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

### Adsorptionsmittel

Adsorptionsmittel werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb^{®}).

### Trocknung

Im Isobuten-haltigen Kohlenwasserstoffgemisch gegebenenfalls enthaltenes Wasser, das beispielsweise aus der Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C₄-Kohlenwasserstoffen ausgenutzt werden oder es können Schleppmittel zugesetzt werden.

Die Trocknung des Kohlenwasserstoffgemisches kann aus diversen Gründen vorteilhaft sein, beispielsweise zur Verringerung der Bildung von Alkoholen (hauptsächlich tert.-Butylalkohol) in Schritt a) bei den Ausführungsformen 2 und 3, zur Verhinderung einer (ungesteuerten) Wassermoderation in der Butenoligomerisierung (Ausführungsform 3 des Schritts a), zur Vermeidung von technischen Problemen durch Abscheidung von Wasser oder zur Vermeidung von Eisbildung bei niedrigen Temperaturen (z. B. Zwischenlagerung).

### Destillation

Destillationsschritte können beispielsweise genutzt werden, um Verunreinigungen abzutrennen (beispielsweise Leichtsieder wie C₃-Kohlenwasserstoffe, Schwersieder wie C₅-Kohlenwasserstoffe) oder um Fraktionen mit unterschiedlichen Isobutenkonzentrationen zu erhalten. Dies kann sowohl direkt mit dem Raffinat I bzw. dem HCC₄ erfolgen oder nachdem eine oder mehrere andere Prozessstufen durchlaufen wurden. Durch direkte Destillation des Raffinats I bzw. des HCC₄s ist beispielsweise eine Trennung in eine an 2-Butenen und n-Butan verarmte, Isobuten reichere Fraktion möglich.

Je nach Zusammensetzung des einzusetzenden technischen Kohlenwasserstoffgemisches und/oder nach den Reinheiten der Zielprodukte kann das technische Kohlenwasserstoffgemisch also direkt in den Schritt a) des erfindungsgemäßen Verfahrens oder aber erst nach einer Vorbehandlung durch eines oder mehrere der vorgenannten Verfahren eingesetzt werden.

### Beschreibung der Figuren

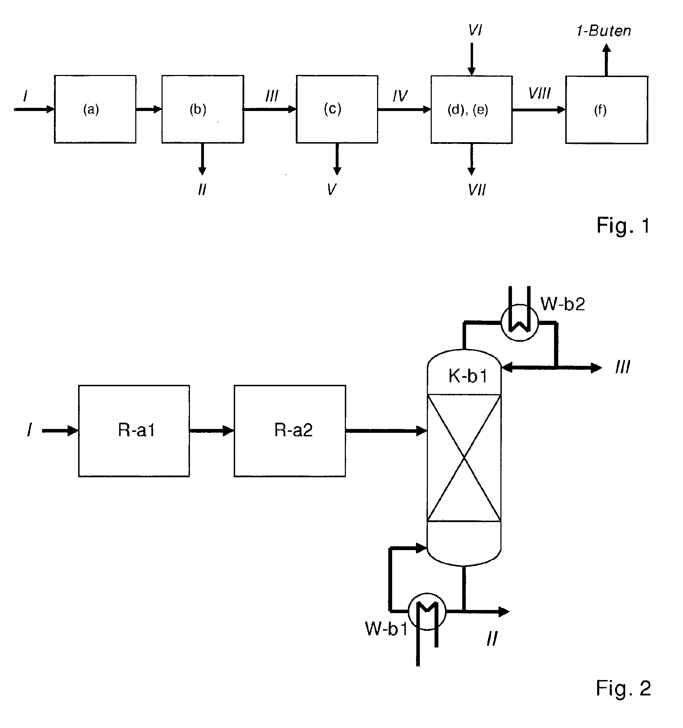

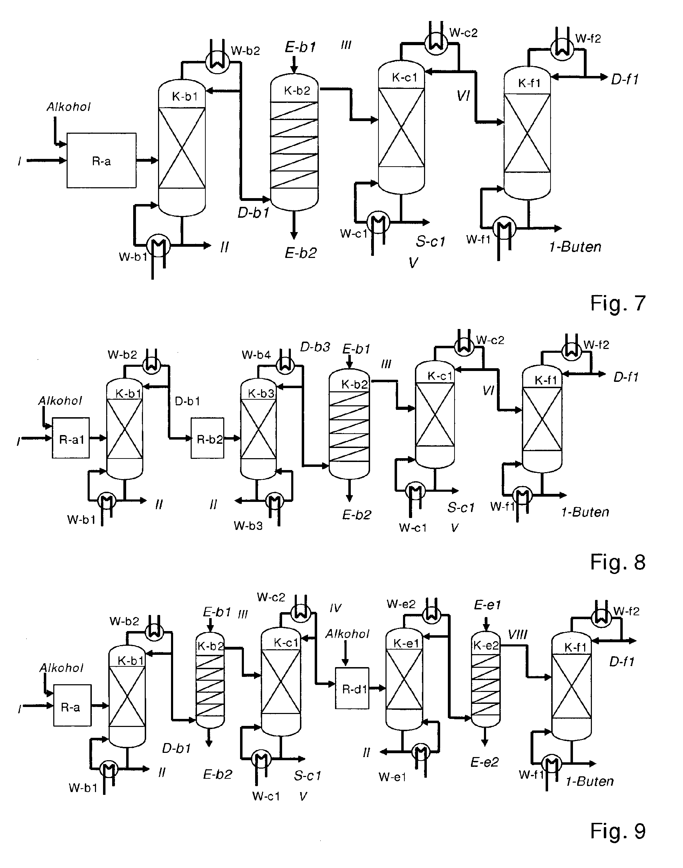

An Hand der Figuren Fig. 1 bis Fig. 6 und Fig. 9 wird das erfindungsgemäße Verfahren nachfolgend näher erläutert, ohne dass das Verfahren auf die dort beispielhaft abgebildeten Ausführungsarten beschränkt sein soll. In den Figuren Fig. 7 und Fig. 8 sind Vergleichsvarianten dargestellt. In den schematischen Darstellungen sind nur die wesentlichen Stufen dargestellt. Auf die Darstellung von verfahrenstechnisch üblichen Strömen, wie z. B. Kühlwasserströmen, Kreislaufströmen, Katalysatorrückführungen oder Rückspeisungen, und/oder üblichen Apparaturen, wie z. B. Wärmetauschern oder Abscheidern wurde teilweise zu Gunsten einer besseren Übersicht verzichtet.
Fig. 1
   Bei dem in Fig.1 schematisch dargestellten Verfahren wird in die Stufe (a) eine technische Mischung von C₄-Kohlenwasserstoffen eingebracht. In der Stufe (a) erfolgt eine teilweise Umsetzung des in der technischen Mischung enthaltenen Isobutens. Die Umsetzung kann z. B. mit Wasser, Alkohol, Formaldehyd oder mit sich selbst erfolgen. Das Produkt der Stufe (a) wird in die Trennstufe (b) überführt, in der nicht umgesetzte C₄-Kohlenwasserstoffe III von den Produkten II vorzugsweise durch thermische Trennverfahren abgetrennt werden. Die nicht umgesetzten C₄-Kohlenwasserstoffe III werden in eine Stufe (c) überführt, die z. B. durch eine einfache Destillationskolonne realisiert sein kann. In dieser Kolonne wird der Strom III in eine Fraktion IV, die Isobuten, Isobutan und 1-Buten aufweist und eine Isobuten-freie oder nahezu Isobuten-freie Fraktion V, die 2-Butene und n-Butane aufweist, getrennt. Die Fraktion IV wird in die zweite Umsetzungsstufe (d) überführt, in welcher das Isobuten mit Alkohol VI zu Alky-tert.-Butylethern (ATBE) umgesetzt wird. In einer anschließenden Trennstufe (e) wird der ATBE VII von nicht umgesetzten Kohlenwasserstoffen VIII getrennt. Diese Kohlenwasserstoffe VIII werden in die Stufe (f) überführt, in welcher das 1-Buten destillativ von den restlichen Kohlenwasserstoffen getrennt wird.
Fig. 2
   In Fig. 2 ist schematisch eine mögliche Ausführungsform der Verfahrensschritte a) und b) dargestellt, wobei die Umsetzung des Isobutens in Stufe (a) die Oligomerisierung des Isobutens ist (Ausführungsform 3). Das technische Gemisch I wird zunächst in einen ersten Oligomerisierungsreaktor R-a1 gefahren. Das Produkt aus dem ersten Reaktor wird in einen zweiten Oligomerisierungsreaktor R-a2 gefahren (Fahrweise mit gleicher oder unterschiedlicher Temperatur etc. möglich). Der Austrag aus dem zweiten Oligomerisierungsreaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Kopfprodukt wird der Strom III abgenommen, der nicht umgesetzte C₄-Kohlenwasserstoffe aufweist, und als Sumpfprodukt wird das Produkt II aus der Umsetzung des Isobutens erhalten, welches hauptsächlich aus Di- und Trimeren des Isobutens besteht.
Fig. 3

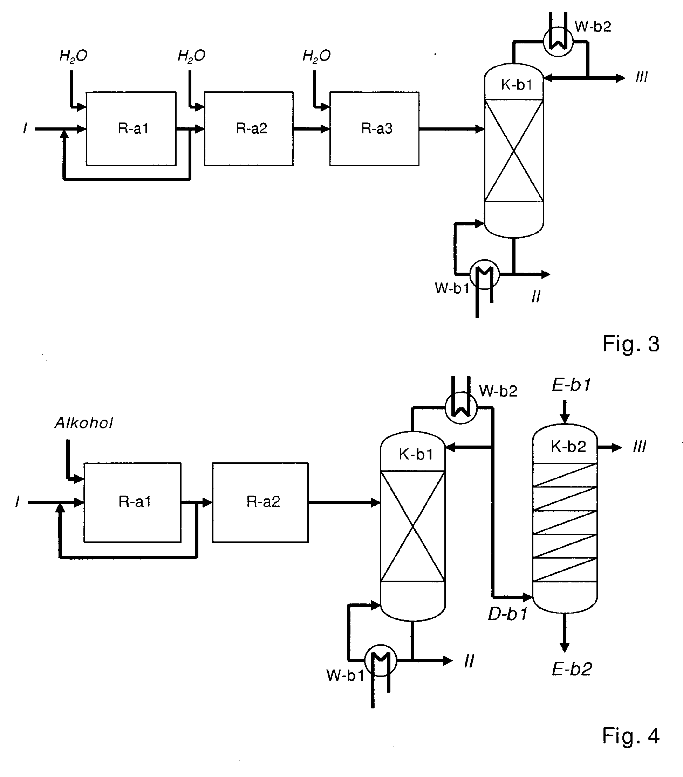

   In Fig. 3 ist schematisch eine mögliche Ausführungsform der Verfahrensschritte a) und b) dargestellt, wobei die Umsetzung des Isobutens in Stufe (a) die Synthese von tert.-Butylalkohol (TBA) ist (Ausführungsform 1). Das technische Gemisch I wird in den ersten Reaktor R-a1 einer Kaskade von drei Reaktoren eingespeist, in den auch Wasser gefahren wird. Der Reaktor R-a1 weist eine Rückführung auf mit der ein Teil des Reaktoraustrags in den Zustrom zum Reaktor zurückgefahren werden kann. Der andere Teil des Reaktoraustrags aus dem ersten Reaktor wird in den zweiten Reaktor R-a2 gefahren, in den ebenfalls Wasser gefahren wird. Der Reaktoraustrag aus dem zweiten Reaktor wird in den dritten Reaktor R-a3 gefahren, in den ebenfalls Wasser eingespeist wird. Der Austrag aus dem dritten Reaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Kopfprodukt wird der Strom III abgenommen, der nicht umgesetzte C₄-Kohlenwasserstoffe aufweist, und als Sumpfprodukt wird das Produkt II, hauptsächlich tert.-Butanol aus der Umsetzung des Isobutens mit Wasser sowie überschüssiges Wasser, erhalten.
Fig. 4
   In Fig. 4 ist schematisch eine mögliche Ausführungsform der Verfahrensschritte a) und b) dargestellt, wobei die Umsetzung des Isobutens in Stufe (a) die Synthese von Alkyl-tert.-butylether (ATBE) ist (Ausführungsform 2). Das technische Gemisch I wird in den ersten Reaktor R-a1 einer Kaskade von zwei Reaktoren eingespeist, in den auch Alkohol eingespeist wird. Der Reaktor R-a1 weist eine Rückführung auf mit der ein Teil des Reaktoraustrags in den Zustrom zum Reaktor zurückgefahren werden kann. Der andere Teil des Reaktoraustrags aus dem ersten Reaktor wird in den zweiten Reaktor R-a2 gefahren. Der Austrag aus dem zweiten Reaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Sumpfprodukt wird das Produkt II, hauptsächlich tert.-Butylether aus der Umsetzung des Isobutens mit Alkohol und gegebenenfalls Restmengen an Alkohol erhalten. Als Kopfprodukt wird der Strom D-b1 abgenommen, der nicht umgesetzte Kohlenwasserstoffe und gegebenenfalls noch Alkohol aufweist. Enthält der Strom Alkohol, was beispielsweise bei Einsatz von Methanol und Ethanol der Fall ist, wird dieser Strom unten in eine Extraktionskolonne K-b2 gefahren, in die im Gegenstrom ein Extraktionsmittel, wie z. B. Wasser, über den am Kopf befindlichen Zulauf E-b1 eingespeist wird, welches über den Ablauf E-b2 am Sumpf der Kolonne entnommen wird. Am Kopf der Kolonne wird als Produkt der Extraktion der Strom aus in Stufe (a) nicht umgesetzten Kohlenwasserstoffen III erhalten.
Fig. 5

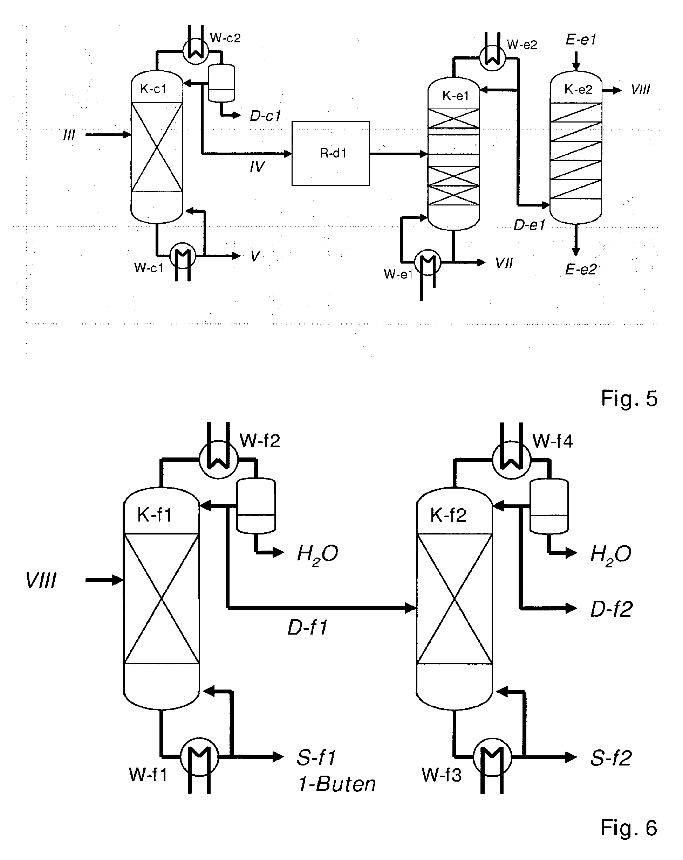

   In Fig. 5 wird eine mögliche Ausführungsform der Stufen c), d) und e) dargestellt. In eine Destillationskolonne K-c1, die mit einem Sumpfverdampfer W-c1 und am Kopf mit einem Kondensator W-c2 und einem Dekanter ausgerüstet ist, wird der Kohlenwasserstoffstrom III aus Stufe b) eingespeist und in eine (nahezu) Isobuten-freie, 2-Butene und n-Butane aufweisende Fraktion V, die am Sumpf der Kolonne abgenommen wird, und eine Isobuten und 1-Buten aufweisende, nahezu n-Butan und 2-Butene freie Fraktion IV, die gegebenenfalls in einem Dekanter von einer wässrigen Phase D-c1 getrennt wird, aufgetrennt. Der Kopf der Kolonne ist so ausgerüstet, dass ein Teil als Rücklauf in die Kolonne zurückgefahren werden kann. Die Fraktion IV wird in den Reaktor R-d1 überführt, in welchen außerdem Alkohol eingespeist wird und in der Fraktion IV enthaltenes Isobuten zu ATBE umgesetzt wird (Stufe d)). Der Austrag aus dem Reaktor R-d1 wird in eine Kolonne K-e1 eingespeist, die als einfache Destillationskolonne oder wie hier dargestellt als Reaktivkolonne ausgeführt sein kann. Die Zuführung des Austrags aus dem Reaktor erfolgt in die Reaktivdestillationskolonne K-e1 vorzugsweise unterhalb der reaktiven Packung. Die Kolonne K-e1 ist mit einem Sumpfverdampfer W-e1 und einem Kondensator W-e2 für das Kopfprodukt ausgerüstet. Als Sumpfprodukt der Kolonne K-e1 wird ATBE erhalten. Das Kopfprodukt D-e1 kann teilweise als Rücklauf in die Kolonne zurückgefahren werden. Der andere Teil wird in die Extraktionskolonne K-e2 überführt, in die im Gegenstrom ein Extraktionsmittel, wie z. B. Wasser, über den am Kopf befindlichen Zulauf E-e1 eingespeist wird, welches über den Ablauf E-e2 am Sumpf der Kolonne entnommen wird. Am Kopf der Kolonne wird als Produkt der Extraktion der Strom aus in Stufe d) und gegebenenfalls e) nicht umgesetzten Kohlenwasserstoffen VIII erhalten.
Fig. 6
   In Fig. 6 ist schematisch eine mögliche Ausführungsform des Verfahrensschrittes f) dargestellt. Der Kohlenwasserstoffstrom VIII aus Stufe e) wird in eine Destillationskolonne K-f1 eingespeist. Die Kolonne K-f1 ist mit einem Sumpfverdampfer W-f1 und einem Kondensator W-f2 für das Kopfprodukt ausgerüstet. Als Sumpfprodukt der Kolonne K-f1 wird 1-Buten S-f1 erhalten. Das Kopfprodukt D-f1, von dem gegebenenfalls in einem Dekanter Wasser abgetrennt wird, kann teilweise als Rücklauf in die Kolonne zurückgefahren werden. Der andere Teil des Kopfproduktes D-f1 wird in die Destillationskolonne K-f2 überführt. Auch diese Kolonne K-f2 ist mit einem Sumpfverdampfer W-f3 und einem Kondensator W-f4 für das Kopfprodukt ausgerüstet. Als Sumpfprodukt der Kolonne K-f2 wird Isobutan S-f2 erhalten. Das Kopfprodukt D-f2, von dem gegebenenfalls in einem Dekanter Wasser abgetrennt wird, kann teilweise als Rücklauf in die Kolonne zurückgefahren werden. Der andere Teil des Kopfproduktes D-f2, der überwiegend aus Leichtsiedern besteht, kann einer weiteren Nutzung oder einer thermischen Verwertung zugeführt werden.
   Das bei dieser Aufarbeitung erhaltene Isobutan (Strom S-f2) kann noch Anteile an ungesättigten Komponenten, hauptsächlich 1-Buten, enthalten. Diese können in einer nachgeschalteten Hydrierung zu den entsprechenden Alkanen hydriert werde. Diese Hydrierung erfolgt nach bekannten Verfahren der Technik, bevorzugt in der flüssigen Phase an einem Palladiumkatalysator. Optional kann diese Hydrierung auch vor der Kolonne K-f2 erfolgen; der Strom D-f1 wird in diesem Fall erst der Hydrierung (in Figur 6 nicht dargestellt) und anschließend der Kolonne K-f2 zugeführt.
Fig. 7
   In dieser Figur ist die im Vergleichsbeispiel berechnete Variante A eines einstufigen Prozesses dargestellt. Bei dieser Variante werden die Stufen (a) und (b) in einer wie in Fig. 4 dargestellten Verschaltung durchgeführt, wobei ein Reaktorsystem R-a an Stelle der Reaktoren R-a1 und R-a2 vorhanden ist. Das aus der Extraktionskolonne K-b2 erhaltene Produkt III wird in die Destillationskolonne K-c1 überführt, in welcher Isobutan, Isobuten und 1-Buten über Kopf abgetrennt werden. Als Sumpfprodukt S-c1 wird eine (nahezu) Isobuten-freie, 2-Butene und n-Butane aufweisende Fraktion V erhalten. Das Destillat VI der Kolonne K-c1 wird direkt in eine weitere Kolonne K-f1 überführt, in welcher es in ein 1-Buten enthaltendes Sumpfprodukt und ein Isobutan und/oder Leichtsieder aufweisendes Kopfprodukt getrennt wird. Als Sumpfprodukt wird eine 1-Buten-reiche Fraktion erhalten, die allerdings den größten Teil des in R-a nicht umgesetzten Isobutens enthält.
Fig. 8
   In dieser Figur ist die im Vergleichsbeispiel berechnete Variante B eines zweistufigen Prozesses dargestellt. Bei dieser Variante werden die Stufen (a) und (b) in einer wie in Fig. 3 dargestellten Verschaltung durchgeführt, wobei ein Reaktor R-a1 an Stelle der Reaktoren R-a1, R-a2 und R-a3 vorhanden ist. Das aus der Kolonne K-b1 erhaltene Destillat D-b1 wird direkt in einen zweiten Reaktor R-b2 gefahren, in dem das im Destillat D-b1 vorhandene restliche Isobuten mit dem ebenfalls vorhandenen Alkohol umgesetzt wird. Das Reaktionsprodukt aus dem Reaktor R-b2 wird in eine Kolonne K-b3 gefahren, in der der in R-b2 gebildete Ether vom restlichen C₄-Kohlenwasserstoffstrom D-b3 als Sumpfprodukt VII abgetrennt wird. Die weitere Aufarbeitung des Destillats D-b3 erfolgt wie in Fig. 7 für das Destillat D-b 1 dargestellt.
Fig. 9
   Zum besseren Vergleich der Verschaltung gemäß der Ausführungsform des erfindungsgemäßen Verfahrens, wie sie im Beispiel eingesetzt wurde, mit den Vergleichsvarianten gemäß den Fig. 7 und 8 ist in Fig. 9 schematisch eine Verschaltung dargestellt, bei der sowohl in Stufe (a) als auch in Stufe (d) ein Veretherungsschritt durchgeführt wird. Die Stufen (a) und (b) werden in einer wie in Fig. 4 dargestellten Verschaltung durchgeführt, wobei ein Reaktorsystem R-a an Stelle der Reaktoren R-a1 und R-a2 vorhanden ist. Die Stufen (c), (d) und (e) werden wie in Fig. 5 beschrieben durchgeführt. Das Produkt VIII, welches aus der Extraktionskolonne K-e2 erhalten wird, wird in die Destillationskolonne K-f1 geführt, in welcher es in ein 1-Buten enthaltendes Sumpfprodukt und ein Isobutan und/oder Leichtsieder aufweisendes Kopfprodukt getrennt wird.
   Die folgenden Beispiele sollen die Erfindung erläutern, ohne den Schutzbereich der sich aus den Patentansprüchen und der Beschreibung ergibt, einzuschränken.

### Beispiele

Die nachfolgenden Beispielrechnungen wurden mit dem Simulationsprogramm ASPEN Plus durchgeführt. Um transparente, reproduzierbare Daten zu erzeugen, wurden nur allgemein zugängliche Stoffdaten eingesetzt. Auf den Einsatz kinetischer Ansätze wurde bewusst verzichtet. Außerdem wurde bei allen Varianten auf den Einsatz einer Reaktivdestillation verzichtet. Durch diese Vereinfachungen ist es dem Fachmann leicht möglich, die Berechnungen nachzuvollziehen. Die eingesetzten Methoden besitzen zwar keine ausreichende Genauigkeit für die Auslegung technischer Anlagen, die qualitativen Unterschiede der Schaltungen werden aber korrekt erfasst. In allen gezeigten Varianten konnte der Isobuten-Umsatz durch Einsatz einer oder mehrerer Reaktivdestillation(en) erhöht werden.

Die Reaktoren und MTBE-Kolonnen wurden mit der Property-Methode "UNIFAC-DMD" berechnet. Für die Berechnung der C₄-Kolonnen wurde mit der Property-Methode "Peng-Robinson" eine Zustandsgleichung eingesetzt. Folgende Annahmen wurden getroffen:
- Alle Reaktoren erreichten bei 50 °C vollständig das mit UNIFAC berechnete Gleichgewicht.
- Die Kolonnen wurden mit einem Rücklaufverhältnis von 0,8 berechnet.
- In den MTBE-Kolonnen wurde über Kopf ein C₄-/Methanol-Azeotrop abgetrennt. Das MeOH wurde in Extraktoren mit Wasser ausgewaschen, die als einfache Komponenten-Splitter modelliert wurden.
- Das aus den Extraktoren erhaltene MeOH-Wasser-Gemisch wurde in einer weiteren Kolonne K-MeOH destillativ aufgearbeitet, die in den Schaltbildern nicht dargestellt wurde. Beide Produkte der K-MeOH wurden in den Prozess rezirkuliert.

Den berechneten Beispielen wurde ein Rohstoffmix aus typischen, auf dem Markt erhältlichen C₄-Rohstoffen zugrundegelegt. Der Rohstoffstrom von 10 t/h enthielt 28 Massen-% Isobuten und 35 Massen-% 1-Buten. Aus diesem Strom sollte das Isobuten durch MTBE-Synthese chemisch abgetrennt und eine 1-Buten-Menge von 3 t/h mit einer Reinheit größer 99,6 % hergestellt werden. Dies entsprach einer 1-Buten-Ausbeute von ca. 85 %. Im 1-Buten Produkt sollten maximal 2000 ppm Isobuten vorliegen. In Tabelle 2 wird die Zusammensetzung des C₄-Rohstoffstroms der angestrebten 1-Buten Spezifikation gegenübergestellt. Die in den Beispielen eingesetzte Menge an Methanol betrug 1900 kg/h (ca. 19 % Überschuss).

**Tabelle 2: Zusammensetzung des C₄-Rohstoffstroms und gewünschte Spezifikation des 1-Butens (%-Angaben sind Massen-%)**

| Komponente | C₄-Feed | | 1-Buten | |
|---|---|---|---|---|
| | [kg/h] | [%] | [kg/h] | [%] |
| Isobutan | 500 | 5,0 | 3 | 0,1 |
| 1-Buten | 3500 | 35,0 | 2990 | 99,7 |
| cis-2-Buten | 1400 | 14,0 | | 0,0 |
| trans-2-Buten | 800 | 8,0 | | 0,0 |
| Isobuten | 2800 | 28,0 | 6 | 0,2 |
| n-Butan | 1000 | 10,0 | 1 | 0,0 |
| Summe | 10000 | 100 | 3000 | 100 |

Nachfolgend wurden drei zur Lösung der Aufgabenstellung unterschiedlich gut geeignete Verfahrensvarianten berechnet.

Die einfachste Variante A war ein einstufiger Prozess, der als Vergleichmaßstab dienen soll. Nach Fig. 7 wurden Methanol und Isobuten in einer Reaktionsstufe R-a bis ins Gleichgewicht umgesetzt. In der Destillationsstufe K-b1 wurde das MTBE (II) als Sumpfprodukt abgetrennt. Die Kolonne hatte 50 theoretische Stufen und wurde bei einem Rücklaufverhältnis von 0,8 betrieben. Das Destillat dieser Kolonne war ein C₄/MeOH-Azeotrop, aus dem das MeOH z. B. mit Wasser in einer Extraktionskolonne K-b2 ausgewaschen werden konnte. Das Raffinat der Extraktionskolonne K-b2 wurde einer C₄-Kolonne analog K-c1 in Fig. 5 zugeführt, in der Isobutan, Isobuten und 1-Buten über Kopf abgetrennt wurden. Das Destillat IV der K-c1 wurde direkt in eine weitere Kolonne K-f1 nach Fig. 6 geleitet, in der vornehmlich Isobutan über Kopf abgetrennt wurde. Als Sumpfprodukt wurde eine 1-Buten-reiche Fraktion erhalten, die den größten Teil des in R-a nicht umgesetzten Isobutens enthielt.

Das mit Variante A dargestellte 1-Buten enthielt 1,9 Massen-% Isobuten, siehe Tabelle 3, und erreichte somit die Zielvorgabe von 2000 ppm nicht. Es sind weitere Maßnahmen zur Erhöhung des Umsatzes notwendig.

Eine andere Prozessverbesserung wurde als Variante B untersucht und ist in Fig. 8 dargestellt. Um das Gleichgewicht weiter in Richtung MTBE zu treiben, wurde hinter die Kolonne K-b1 ein weiterer Reaktor R-b2 geschaltet, der das Rest-Isobuten mit dem in K-b1 als Azeotrop D-b1 über Kopf abgetrennten MeOH umsetzte. Das gebildete MTBE wurde in einer weiteren C₄-/MTBE-Destillation K-b3 abgetrennt. Dabei musste der gesamte C₄-Strom ein zweites mal über Kopf destilliert werden. Der Energiebedarf der K-b3 war also nahezu gleich groß, wie der der K-b1. Anschließend wurde die Extraktion K-b2 und die 1-Buten-Destillation K-c1 und K-f1 wie in Variante A durchfahren. Nun erreichte das 1-Buten-Produkt mit weniger als 500 ppm Isobuten die geforderte Produktspezifikation. Der Gesamtenergiebedarf der Anlage ist gegenüber Variante A allerdings um etwa 12 % höher (siehe Tabelle 4).

Im erfindungsgemäßen Verfahren Variante C nach Fig. 9 wurde eine zweite Reaktions-, Destillations- und Extraktionsstufe R-d, K-e1 und K-e2 zwischen die beiden C₄-Kolonnen K-c1 und K-f1 geschaltet. Dies hatte den Vorteil, dass der größere Teile des Zulaufs III der K-c1 als Sumpfprodukt V anfällt und nur der Teil des C₄-Stromes ein zweites mal destilliert wurde, der in K-f1 zu reinem 1-Buten aufgearbeitet werden sollte. Im vorliegenden Fall war der Energiebedarf der K-b2 bei Variante B mehr als doppelt so hoch, wie der Energiebedarf der K-e1 bei Variante C. Nachteilig an Variante C war, dass eine zweite Extraktionskolonne K-e2 gebaut werden muss. Da der Durchsatz durch R-d1, K-e1 und K-e2 aber weniger als die Hälfte des Durchsatzes durch K-b3 bei Variante B betrug, fielen die Gesamtinvestitionskosten entsprechend niedriger aus. Die Isobutenmenge im 1-Buten-Produkt lag im berechneten Beispiel ebenfalls bei weniger als 500 ppm.

Tabelle 3 zeigt die erreichten Umsätze der drei Varianten. Während Variante A die geforderte Qualität des 1-Buten-Produktes klar verfehlte, wurde in den beiden zweistufigen Verfahren B und C ein spezifikationsgerechtes Produkt berechnet. Der erhöhte Isobuten-Umsatz wurde bei beiden Varianten durch destillative Abtrennung des Reaktionsproduktes MTBE vor einer zweiten Reaktionsstufe und damit durch erhöhten Energieeinsatz erreicht. Durch die erfindungsgemäße Anordnung der zweiten Reaktionsstufe in Variante C zwischen den beiden C₄-Kolonnen K-c1 und K-f1 wurde die Menge des zusätzlich zu destillierenden Stromes jedoch auf weniger als die Hälfte reduziert. Dies führt zu deutlichen Einsparungen bei Energiebedarf und Investment.

**Tabelle 3: Umsätze und 1-Buten-Qualitäten der drei Varianten**

| | Variante A, einstufig | Variante B, zweistufig | Variante C, zweistufig |
|---|---|---|---|
| Isobuten im Feed [kg/h] | 2800 | 2800 | 2800 |
| Isobuten nach Stufe 1 [kg/h] | 65,6 | 65,6 | 65,6 |
| Umsatz Stufe 1 [%] | 97,7 | 97,7 | 97,7 |
| Isobuten nach Stufe 2 [kg/h] | 0,0 | 0,9 | 0,9 |
| Umsatz Stufe 2 [%] | 0,0 | 98,6 | 98,6 |
| Umsatz Gesamt [%] | 97,7 | 99,97 | 99,97 |
| Isobuten im 1-Buten [ppm] | 19130 | < 500 | < 500 |
| Reinheit 1-Buten [%] | 97,8 | 99,7 | 99,7 |

In Tabelle 4 sind die berechneten Energiebedarfszahlen aller drei Varianten zusammengestellt. In allen Schaltungen ist der Energiebedarf der Kolonnen K-b1, K-c1 und K-f1 nahezu identisch. Variante A hatte zwar den niedrigsten Gesamtenergiebedarf, die Produktspezifikation wurde jedoch verfehlt. Bei Variante B musste im MTBE-Teil die doppelte C₄-Menge über Kopf destilliert werden, ihr Energiebedarf lag daher um 12 % höher als Bei Variante A. Demgegenüber zeigte Variante C einen Weg, den geforderten Umsatz bei nur etwa 6 % erhöhtem Energiebedarf zu erreichen.

**Tabelle 4: Energiebedarf der drei Varianten**

| | Variante A, einstufig | Variante B, zweistufig | Variante C, zweistufig |
|---|---|---|---|
| Q K-b1 [kW] | 1353 | 1353 | 1353 |
| Q K-b3 [kW] | 254 | 233 | 328 |
| Q K-c1 [kW] | 3530 | 3493 | 3530 |
| Q K-b2 [kW] | | 1333 | 620 |
| Q K-e1 [kW] | | | |
| Q K-f1 [kW | 3741 | 3672 | 3669 |
| Q MTBE [kW] | 1608 | 2919 | 2300 |
| Q Gesamt [kW] | 8878 | 10084 | 9498 |
| Mehrbedarf ΔQ MTBE [%] | 0 | 81,6 | 43,1 |
| Mehrbedarf ΔQ Gesamt [%] | 0 | 12,0 | 6,5 |

Die Bezeichnungen in den Figuren Fig. 1 bis Fig. 9 haben folgende Bedeutungen:
- (a): Teilumsetzung Isobuten zu Produkten II
- (b): Abtrennung C₄-Kohlenwasserstoffe III
- (c): destillative Trennung von III in IV und V
- (d): Veretherung von Isobuten mit Alkohol VI
- (e): Abtrennung von t-Butylether VII
- (f): Abtrennung 1-Buten

- I: technische Mischung von C₄-Kohlenwasserstoffen
- II: Produkte aus der Isobuten-Umsetzung
- III: verbleibende C₄-Kohlenwasserstoffe
- IV: 1-Buten und Isobuten-haltige Fraktion
- V: Isobuten-freie, 2-Butene und n-Butane aufweisende Fraktion
- VI: Alkohol
- VII: Alkyl-tert.-Butylether
- VIII: C₄-Kohlenwasserstoffe aus Stufe e)

- D-b1: Destillat der K-b 1
- D-b3: Destillat der K-b3
- D-c1: bei der Kondensation am Kopf der K-c1 anfallende wässrige Phase
- D-e1: Kopfprodukt von K-e1
- D-f1: Destillat K-f1, organische Phase
- D-f2: Leichtsieder
- E-b1: Zulauf Extraktionsmittel
- E-b2: Ablauf Extraktionsmittel
- E-e1: Zulauf Extraktionsmittel
- E-e2: Ablauf Extraktionsmittel
- K-b1: Destillation
- K-b2: Extraktionskolonne
- K-b3: Destillation
- K-c1: Kolonne zur Trennung der C₄-Kohlenwasserstoffe
- K-e1: Kolonne zur Abtrennung des Ethers
- K-e2: Extraktionskolonne
- K-f1: Kolonne zur 1-Buten Abtrennung
- K-f2: Kolonne Isobutan-Abtrennung
- R-a: Reaktor
- R-a1: Reaktor
- R-a2: Reaktor
- R-a3: Reaktor
- R-b2: Reaktor Veretherung (Vergleichsbeispiel)
- R-d1: Reaktor Veretherung
- S-f1: 1-Buten
- S-f2: Isobutan
- W-b1: Sumpfverdampfer
- W-b2: Kondensator
- W-b3: Sumpfverdampfer
- W-b4: Kondensator
- W-c1: Sumpfverdampfer
- W-c2: Kondensator
- W-e1: Sumpfverdampfer
- W-e2: Kondensator
- W-f1: Sumpfverdampfer
- W-f2: Kondensator
- W-f3: Sumpfverdampfer
- W-f4: Kondensator

## Patentansprüche

1. Verfahren zur Herstellung von 1-Buten aus technischen Mischungen von C₄-Kohlenwasserstoffen 1, die mindestens 1-Buten, Isobuten, n-Butan und 2-Butene enthalten, **gekennzeichnet durch** die Verfahrensschritte
a) Umsetzung von Teilen des in der technischen Mischung enthaltenen Isobutens zu bei Normaldruck höher als 30 °C siedenden Produkten II,
b) Abtrennung der nicht umgesetzten C₄-Kohlenwasserstoffe III aus dem Austrag der Stufe a) **durch** thermische Trennverfahren,
c) destillative Trennung der C₄-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) Umsetzung des in Fraktion IV enthaltenen Isobutens mit einem Alkohol VI in Gegenwart saurer Katalysatoren zu tert.-Butylethern VII,
e) Abtrennung der nicht umgesetzten C₄-Kohlenwasserstoffe VIII aus dem Austrag von Stufe d) und
f) destillative Abtrennung des 1-Butens aus den in e) erhaltenen C₄-Kohlenwasserstoffen VIII.

2. Verfahren nach Anspruch 1,
**dadurch gekennzeichnet,**
**dass** die sauer katalysierte Veretherung in Stufe d) so durchgeführt wird, dass zumindest eine Reaktionsstufe als Reaktivdestillation durchgeführt wird.

3. Verfahren nach Anspruch 1 oder 2,
**dadurch gekennzeichnet,**
**dass** die sauer katalysierte Veretherung in Stufe d) in mindestens zwei Reaktionsstufen durchgeführt wird, wobei mindestens die letzte Reaktionsstufe als Reaktivdestillation durchgeführt wird.

4. Verfahren nach zumindest einem der Ansprüche 1 bis 3,
**dadurch gekennzeichnet,**
**dass** in Stufe d) als Alkohol Methanol oder Ethanol in der Veretherung eingesetzt wird.

5. Verfahren nach zumindest einem der Ansprüche 1 bis 4,
**dadurch gekennzeichnet,**
**dass** in Stufe e) Restmengen an Alkohol in den C₄-Kohlenwasserstoffen in einem Extraktionsschritt mit Wasser ausgewaschen werden.

6. Verfahren nach zumindest einem der Ansprüche 1 bis 5,
**dadurch gekennzeichnet,**
**dass** in den C₄-Kohlenwasserstoffströmen enthaltene mehrfach ungesättigten Kohlenwasserstoffe in einer zusätzlichen Reinigungsstufe, die einer oder mehreren der Verfahrensstufen a), b), c) oder d) vorgeschaltet wird, katalytisch hydriert werden.

7. Verfahren nach Anspruch 6,
**dadurch gekennzeichnet,**
**dass** die Hydrierung der mehrfach ungesättigten Verbindungen in mindestens zwei Reaktionsstufen erfolgt, wobei mindestens die letzte Reaktionsstufe in Anwesenheit von 0,05 bis 100 wppm CO durchgeführt wird.

8. Verfahren nach zumindest einem der Ansprüche 1 bis 7,
**dadurch gekennzeichnet,**
**dass** der Umsatz des Isobutens in der Verfahrensstufe a) über 70 % beträgt.

9. Verfahren nach Anspruch 1 bis 4,
**dadurch gekennzeichnet,**
**dass** die Umsetzung des Isobutens in der Verfahrensstufe a) mit Wasser oder Alkohol als Reaktionspartner durchgeführt wird und der Umsatz an Isobuten über 75 % beträgt.

10. Verfahren nach zumindest einem der Ansprüche 1 bis 9,
**dadurch gekennzeichnet,**
**dass** in Stufe a) Isobuten unter saurer Katalyse mit Alkoholen zu Alkyl-tert.-Butylethern (ATBE) oder mit Wasser zu tertiärem Butylalkohol (TBA) umgesetzt wird.

11. Verfahren nach Anspruch 10,
**dadurch gekennzeichnet,**
**dass** bei der Umsetzung von Isobuten mit Wasser oder Alkohol als saurer Katalysator ein lonentauscherharz eingesetzt wird.

12. Verfahren nach zumindest einem der Ansprüche 10 oder 11,
**dadurch gekennzeichnet,**
**dass** als Alkohol Methanol oder Ethanol eingesetzt wird.

13. Verfahren nach einem der Ansprüche 10 bis 12,
**dadurch gekennzeichnet,**
**dass** wenn in Stufe a) Isobuten mit Alkohol umgesetzt wurde, in Stufe b) in einem ersten Schritt C₄-Kohlenwasserstoffe zusammen mit Restmengen Alkohol vom Produkt II destillativ abgetrennt werden und in einem zweiten Schritt der Alkohol extraktiv von den C₄-Kohlenwasserstoffen III abgetrennt wird.

14. Verfahren nach zumindest einem der Ansprüche 10 bis 13,
**dadurch gekennzeichnet,**
**dass** in Stufe a) und in Stufe d) der gleiche Alkohol eingesetzt wird.

15. Verfahren nach Anspruch 14,
**dadurch gekennzeichnet,**
**dass** die in Verfahrensschritt e) bei der Abtrennung der C₄-Kohlenwasserstoffe VIII erhaltenen tert.-Butylether VIII zusammen mit überschüssigem Alkohol ganz oder teilweise in Schritt a) und/oder b) zurückgeführt werden.

16. Verfahren nach einem der Ansprüche 10 oder 11,
**dadurch gekennzeichnet,**
**dass** wenn in Stufe a) Isobuten mit Wasser umgesetzt wurde, in Stufe b) die C₄-Kohlenwasserstoffe destillativ von den Produkten II abgetrennt werden.

17. Verfahren nach Anspruch 16,
**dadurch gekennzeichnet,**
**dass** wenn in Stufe a) Isobuten mit Wasser umgesetzt wurde, in Stufe b) die C₄-Kohlenwasserstoffe zusammen mit etwas Wasser vom TBA-/Wasser-Gemisch abgetrennt wird.

18. Verfahren nach zumindest einem der Ansprüche 1 bis 8,
**dadurch gekennzeichnet,**
**dass** in Stufe a) das Isobuten zu Diisobuten umgesetzt wird.

19. Verfahren nach Anspruch 18,
**dadurch gekennzeichnet,**
**dass** die Umsetzung in Stufe a) unter saurer Katalyse erfolgt.

20. Verfahren nach Anspruch 18 oder 19,
**dadurch gekennzeichnet,**
**dass** die Umsetzung in Stufe a) in Gegenwart eines Kationentauschers erfolgt.

21. Verfahren nach zumindest einem der Ansprüche 18 bis 20
**dadurch gekennzeichnet,**
**dass** ein Kationenaustauscher eingesetzt wird, dessen acide Wasserstoffatome teilweise gegen Metallionen der 1. bis 12. Gruppe des Periodensystems ausgetauscht wurden.

22. Verfahren nach Anspruch 21,
**dadurch gekennzeichnet,**
**dass** 1 bis 70 % der aciden Wasserstoffatome des in Stufe a) verwendeten Ionenaustauschers gegen Metallionen ausgetauscht sind.

23. Verfahren nach zumindest einem der Ansprüche 18 bis 22,
**dadurch gekennzeichnet,**
**dass** in Stufe b) die C₄-Kohlenwasserstoffe III destillativ von den Produkten II abgetrennt werden.

24. Verfahren nach zumindest einem der Ansprüche 1 bis 8,
**dadurch gekennzeichnet,**
**dass** in Stufe a) das Isobuten mit Formaldehyd zu 4,4-Dimethyl-1,3-Dioxan oder 3-Methyl-3-buten-1-ol umgesetzt wird.

25. Verfahren nach zumindest einem der Ansprüche 1 bis 24,
**dadurch gekennzeichnet,**
**dass** 1-Buten mit einem Gehalt an Isobuten von kleiner 5000 wppm erhalten wird.

## Claims

1. Process for preparing 1-butene from technical mixtures of C₄ hydrocarbons I which comprise at least 1-butene, isobutene, n-butane and 2-butenes, **characterized by** the process steps of
a) reacting portions of the isobutene present in the technical mixture to give products II which boil at higher than 30°C at standard pressure,
b) removing the unconverted C₄ hydrocarbons III from the effluent of stage a) by thermal separation processes,
c) distillatively separating the C₄ hydrocarbons III into a fraction IV comprising at least 1-butene and isobutene, and a virtually isobutene-free fraction V comprising at least 2-butenes and n-butane,
d) reacting the isobutene present in fraction IV with an alcohol VI in the presence of acidic catalysts to give tert-butyl ethers VII,
e) removing the unconverted C₄ hydrocarbons VIII from the effluent of stage d) and
f) distillatively removing the 1-butene from the C₄ hydrocarbons VIII obtained in e).

2. Process according to Claim 1,
**characterized in that**
the acid-catalyzed etherification is carried out in stage d) in such a way that at least one reaction stage is carried out as a reactive distillation.

3. Process according to Claim 1 or 2,
**characterized in that**
the acid-catalyzed etherification in stage d) is carried out in at least two reaction stages, at least the last reaction stage being carried out as a reactive distillation.

4. Process according to at least one of Claims 1 to 3,
**characterized in that**
the alcohol used in the etherification in stage d) is methanol or ethanol.

5. Process according to at least one of Claims 1 to 4,
**characterized in that**
the residual amount of alcohol in the C₄ hydrocarbons is scrubbed out in stage e) in an extraction step with water.

6. Process according to at least one of Claims 1 to 5,
**characterized in that**
polyunsaturated hydrocarbons present in the C₄ hydrocarbon streams are hydrogenated catalytically in an additional purification stage which precedes one or more of process stages a), b), c) or d).

7. Process according to Claim 6,
**characterized in that**
the polyunsaturated compounds are hydrogenated in at least two reaction stages, at least the last reaction stage being carried out in the presence of from 0.05 to 100 ppmw of CO.

8. Process according to at least one of Claims 1 to 7,
**characterized in that**
the conversion of the isobutene in process stage a) is over 70%.

9. Process according to any one of Claims 1 to 4,
**characterized in that**
the reaction of the isobutene in process stage a) is carried out with water or alcohol as a reactant and the conversion of isobutene is over 75%.

10. Process according to at least one of Claims 1 to 9,
**characterized in that**
isobutene is reacted in stage a) under acidic catalysis with alcohols to give alkyl tert-butyl ethers (ATBE) or with water to give tertiary butyl alcohol (TBA).

11. Process according to Claim 10,
**characterized in that**
an ion exchange resin is used as the acidic catalyst in the reaction of isobutene with water or alcohol.

12. process according to at least one of Claims 10 and 11,
**characterized in that**
the alcohol used is methanol or ethanol.

13. Process according to any one of Claims 10 to 12,
**characterized in that**,
when isobutene has been reacted with alcohol in stage a), C₄ hydrocarbons, in stage b), are removed by distillation from the product II in a first step together with a residual amount of alcohol, and the alcohol is removed by extraction from the C₄ hydrocarbons III in a second step.

14. Process according to at least one of Claims 10 to 13,
**characterized in that**
the same alcohol is used in stage a) and in stage d).

15. Process according to Claim 14,
**characterized in that**
the tert-butyl ethers VII obtained in process step e) in the course of removal of the C₄ hydrocarbons VIII are returned together with excess alcohol fully or partly into step a) and/or b).

16. Process according to either of Claims 10 and 11,
**characterized in that**,
when isobutene has been reacted with water in stage a), the C₄ hydrocarbons, in stage b), are removed by distillation from the products II.

17. Process according to Claim 16,
**characterized in that**,
when isobutene has been reacted with water in stage a), the C₄ hydrocarbons, in stage b), are removed from the TBA/water mixture together with a little water.

18. Process according to at least one of Claims 1 to 8,
**characterized in that**
the isobutene is converted to diisobutene in stage a).

19. Process according to Claim 18,
**characterized in that**
the reaction in stage a) is effected under acidic catalysis.

20. Process according to either of Claims 18 and 19,
**characterized in that**
the reaction in stage a) is effected in the presence of a cation exchanger.

21. Process according to at least one of Claims 18 to 20,
**characterized in that**
a cation exchanger is used whose acidic hydrogen atoms have been exchanged partly for metal ions of group 1 to 12 of the Periodic Table.

22. Process according to Claim 21,
**characterized in that**
from 1 to 70% of the acidic hydrogen atoms of the ion exchanger used in stage a) have been exchanged for metal ions.

23. Process according to at least one of Claims 18 to 22,
**characterized in that**
the C₄ hydrocarbons III are removed by distillation from the products II in stage b).

24. Process according to at least one of Claims 1 to 8,
**characterized in that**
the isobutene is reacted in stage a) with formaldehyde to give 4,4-dimethyl-1,3-dioxane or 3-methyl-3-buten-1-ol.

25. Process according to at least one of Claims 1 to 24,
**characterized in that**
1-butene is obtained with a content of isobutene of less than 5000 ppmw.

## Revendications

1. Procédé de fabrication de 1-butène à partir de mélanges techniques d'hydrocarbures en C₄ I, contenant au moins du 1-butène, de l'isobutène, du n-butane et des 2-butènes, **caractérisé par** les étapes de procédé :
a) la transformation de parties de l'isobutène contenu dans le mélange technique en produits II d'un point d'ébullition supérieur à 30°C à pression normale,
b) la séparation des hydrocarbures en C₄ non réagis III de la sortie de l'étape a) par des procédés de séparation thermiques,
c) la séparation par distillation des hydrocarbures en C₄ III en une fraction IV contenant au moins du 1-butène et de l'isobutène et une fraction V presque exempte d'isobutène, contenant au moins des 2-butènes et du n-butane,
d) la mise en réaction de l'isobutène contenu dans la fraction IV avec un alcool VI en présence de catalyseurs acides pour obtenir des éthers de tert.-butyle VII,
e) la séparation des hydrocarbures en C₄ non réagis VIII de la sortie de l'étape d) et
f) la séparation par distillation du 1-butène des hydrocarbures en C₄ VIII obtenus en e).

2. Procédé selon la revendication 1, **caractérisé en ce que** l'éthérification sous catalyse acide à l'étape d) est réalisée de manière à ce qu'au moins une étape de réaction soit réalisée sous la forme d'une distillation réactive.

3. Procédé selon la revendication 1 ou 2, **caractérisé en ce que** l'éthérification sous catalyse acide à l'étape d) est réalisée en au moins deux étapes de réaction, au moins la dernière étape de réaction étant réalisée sous la forme d'une distillation réactive.

4. Procédé selon au moins l'une quelconque des revendications 1 à 3, **caractérisé en ce que** du méthanol ou de l'éthanol est utilisé dans l'éthérification à l'étape d) en tant qu'alcool.

5. Procédé selon au moins l'une quelconque des revendications 1 à 4, **caractérisé en ce que** des quantités résiduelles d'alcool dans les hydrocarbures en C₄ sont éliminées par lavage avec de l'eau lors d'une étape d'extraction à l'étape e).

6. Procédé selon au moins l'une quelconque des revendications 1 à 5, **caractérisé en ce que** des hydrocarbures polyinsaturés contenus dans les courants d'hydrocarbures en C₄ sont hydrogénés sous catalyse lors d'une étape de purification supplémentaire, qui est située en amont d'une ou de plusieurs des étapes de procédé a), b), c) ou d).

7. Procédé selon la revendication 6, **caractérisé en ce que** l'hydrogénation des composés polyinsaturés a lieu en au moins deux étapes de réaction, au moins la dernière étape de réaction étant réalisée en présence de 0,05 à 100 ppm en poids de CO.

8. Procédé selon au moins l'une quelconque des revendications 1 à 7, **caractérisé en ce que** la conversion de l'isobutène à l'étape de procédé a) est supérieure à 70 %.

9. Procédé selon les revendications 1 à 4, **caractérisé en ce que** la transformation de l'isobutène à l'étape de procédé a) est réalisée avec de l'eau ou un alcool en tant que partenaire de réaction et la conversion de l'isobutène est supérieure à 75 %.

10. Procédé selon au moins l'une quelconque des revendications 1 à 9, **caractérisé en ce qu'**à l'étape a), l'isobutène est transformé sous catalyse acide avec des alcools en éthers d'alkyle et de tert.-butyle (ATBE) ou avec de l'eau en alcool butylique tertiaire (TBA).

11. Procédé selon la revendication 10, **caractérisé en ce qu'**une résine échangeuse d'ions est utilisée en tant que catalyseur acide lors de la réaction de l'isobutène avec de l'eau ou un alcool.

12. Procédé selon au moins l'une quelconque des revendications 10 ou 11, **caractérisé en ce que** du méthanol ou de l'éthanol est utilisé en tant qu'alcool.

13. Procédé selon au moins l'une quelconque des revendications 10 à 12, **caractérisé en ce que** lorsque de l'isobutène a été mis en réaction avec un alcool à l'étape a), des hydrocarbures en C₄ sont séparés par distillation avec des quantités résiduelles d'alcool du produit II à l'étape b) lors d'une première étape et, lors d'une seconde étape, l'alcool est séparé par extraction des hydrocarbures en C₄ III.

14. Procédé selon au moins l'une quelconque des revendications 10 à 13, **caractérisé en ce que** le même alcool est utilisé à l'étape a) et à l'étape d).

15. Procédé selon la revendication 14, **caractérisé en ce que** l'éther tert.-butylique VII obtenu à l'étape de procédé e) lors de la séparation des hydrocarbures en C₄ VIII est recyclé en totalité ou en partie dans l'étape a) et/ou b) avec l'alcool en excès.

16. Procédé selon l'une quelconque des revendications 10 ou 11, **caractérisé en ce que**, lorsque de l'isobutène a été mis en réaction avec de l'eau à l'étape a), les hydrocarbures en C₄ sont séparés par distillation des produits II à l'étape b).

17. Procédé selon la revendication 16, **caractérisé en ce que**, lorsque de l'isobutène a été mis en réaction avec de l'eau à l'étape a), les hydrocarbures en C₄ sont séparé du mélange TBA/eau avec un peu d'eau à l'étape b).

18. Procédé selon au moins l'une quelconque des revendications 1 à 8, **caractérisé en ce qu'**à l'étape a), l'isobutène est transformé en diisobutène.

19. Procédé selon la revendication 18, **caractérisé en ce que** la réaction à l'étape a) a lieu sous catalyse acide.

20. Procédé selon la revendication 18 ou 19, **caractérisé en ce que** la réaction à l'étape a) a lieu en présence d'un échangeur de cations.

21. Procédé selon au moins l'une quelconque des revendications 18 à 20, **caractérisé en ce qu'**un échangeur de cations dont les atomes d'hydrogène acides ont été remplacés en partie par des ions métalliques des groupes 1 à 12 du tableau périodique est utilisé.

22. Procédé selon la revendication 21, **caractérisé en ce que** 1 à 70 % des atomes d'hydrogène acides de l'échangeur d'ions utilisé à l'étape a) sont remplacés par des ions métalliques.

23. Procédé selon au moins l'une quelconque des revendications 18 à 22, **caractérisé en ce qu'**à l'étape b), les hydrocarbures en C₄ III sont séparés par distillation des produits II.

24. Procédé selon au moins l'une quelconque des revendications 1 à 8, **caractérisé en ce qu'**à l'étape a), l'isobutène est mis en réaction avec du formaldéhyde pour obtenir du 4,4-diméthyl-1,3-dioxane ou du 3-méthyl-3-butén-1-ol.

25. Procédé selon au moins l'une quelconque des revendications 1 à 24, **caractérisé en ce qu'**un 1-butène ayant une teneur en isobutène inférieure à 5 000 ppm en poids est obtenu.
